(19) Europäisches Patentamt
European Patent Office
Office européen des brevets

(11) EP 2 462 898 A1

(12) EUROPEAN PATENT APPLICATION

(43) Date of publication:
**13.06.2012 Bulletin 2012/24**

(21) Application number: **10194425.4**

(22) Date of filing: **09.12.2010**

(51) Int Cl.:
***A61F 2/16*** *(2006.01)*
***C08G 61/12*** *(2006.01)*
***C08K 5/07*** *(2006.01)*
***A61L 27/18*** *(2006.01)*
***A61L 27/48*** *(2006.01)*
*C08L 65/00* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Université de Liège**
**4031 Angleur (BE)**

(72) Inventors:
- **Bozukova, Dimitriya, Dr.**
  **4000 LIEGE (BE)**
- **Jerome, Christine, Prof.**
  **4000 LIEGE (BE)**

(54) **Composite comprising nanoparticles and method for making nanoparticles**

(57) A method for making nanoparticles of a conjugated polymer, the method having the following steps (i) providing a reaction mixture with a monomer, a photosensitising agent and a solvent, and (ii) exposing the reaction mixture to photo irradiation to form nanoparticles of a conjugated polymer. The composite compring the nanoparticles may be used in an intraocular lens implant.

EP 2 462 898 A1

## Description

The present invention relates to a method for making nanoparticles, in particular, nanoparticles for use in a composite. The composite may be a hydrogel. The composite comprising nanoparticles made by the method of the invention may, for example, be for use in an intraocular lens implant.

Heterocyclic conjugated polymers are widely used in the fabrication of biomaterials, such as biosensors, stimuli-responsive drug delivery systems, etc., due to their specific properties, such as electroconductivity, light absorbance, fluorescence, etc. Depending on the particular application, the polymers may be used as films, in solution or in the form of particles. Poly-thiophenes (PTh), including oligothiophenes, are examples of heterocyclic conjugated polymers which are environmentally and thermally stable materials. Such polymers have been used in biomaterials, such as artificial noses and muscles.

For some biomaterial applications, such as stimuli-responsive systems, drug-delivery systems, optical elements and biosensors, PTh in the shape of nanoparticles or mesoparticles has been used. Reference herein to biomaterials is intended to refer to materials, products and substances which can be used in contact with biological samples (tissues, fluids, organs, cells, cell components, etc.) under *in vitro, ex vivo* and *in vivo* conditions.

When used in the preparation of biomaterials, it is preferable for the PTh particles to have a high purity, so that any potentially toxic effects on a biological system it may be subsequently placed in contact with are reduced or eliminated. Furthermore, if PTh is used in nanoparticles, it preferably has well controlled dimensions.

In known methods for producing nanoparticles of conjugated polymers potential contaminants usually result from the synthesis of the conjugated molecules by polymerization of the respective monomer, such as thiophene or its derivatives.

In particular, in the known methods for the production of nanoparticles of polythiophene, and/or derivatives thereof, it is difficult, if not impossible, to control the size and shape of the final particles (Choi et al. Synth. Mater. 2004, 141, 293-299; Landfester et al. Adv. Mater. 2002, 14/9, 651-655; Xia et al. J. Am. Chem. Soc. 2004, 126, 8735-8743). This is a consequence of the method of particle preparation. Some of the methods use metallic nanoparticles (i.e. gold) as templates, that are coated by the conjugated polymer (Zhai, L. et al. J. Mater. Chem. 2004, 14, 141-143); in other methods the monomer is polymerized at the surface of gold nanoparticles using electrochemistry (Si et al. J. Am. Chem. Soc. 2007, 129, 3888-3896) or chemical oxidation (Zhang, Z. et al. Mater. Lett. 2006, 60, 1039-1042); in still further methods particles are produced by precipitation of a pre-formed conjugated polymer (synthesized by chemical oxidation with an $Fe^{3+}$ salt) on the surface of the inorganic nano-cores. Oligothiophene nanoparticles may also be precipitated from a dilute organic solution on the surface of graphite and mica (Surin et al. Synth. Met. 2004, 147, 67-72). The oligomers may then be capped by one or two short hydrophobic poly(ethylene oxide) segments. In yet another example, structurally ordered poly(3,3'-dialkylquarterthiophene) (PQT-12) nanoparticles were prepared by $Fe^{3+}$ - catalyzed oxidation polymerization in dichlorobenzene and used in the preparation of organic thin film transistors (OTFTs) (Ong et al. Adv. Mater. 2005, 17, 1141-1144). Similarly, $Fe^{3+}$ -catalyzed oxidation polymerization was used for the fabrication of nanoparticles of poly(Th) dendrimers (Advincula, Dalton Trans. 2006, 2778-2784) and core/shell poly(styrene/Th) (Lee et al. Current Appl. Phys. 2008, 659-663) and poly(3,4-ethylenedioxythiophene) (PEDOT) nanoparticles (Oh et al. Current Appl. Phys. 2002, 2, 273-277) in water.

In all the aforementioned known methods the nanoparticles produced contain significant amounts of synthetic contaminants making them unsuitable, or less than optimal, for use in biomaterials. An aim of the present invention is therefore to provide nanoparticles of conjugated polymers which have no, or very little, toxic contaminants. It is also an aim to produce particles with controlled dimensions.

According to a first aspect the invention provides a method for the production of nanoparticles comprising a conjugated polymer, wherein the method comprises (i) providing a reaction mixture comprising a monomer, a photosensitising agent and a solvent, and (ii) exposing the reaction mixture to photo irradiation to form nanoparticles of a conjugated polymer.

Preferably a nanoparticle is a particle wherein at least one dimension is equal or lower than about 1000 nm, preferably is equal or lower than about 250 nm and even more preferably is equal or lower than about 100 nm. The one dimension that is equal to or lower than about 1000 nm, preferably is equal or lower than about 250 nm and even more preferably is equal or lower than about 100 nm may be the radius.

Preferably the nanoparticles produced by the method of the invention are substantially spherical.

The solvent may be any suitable solvent. The solvent may be water, methanol, ethanol, iso-propanol, tetrahydrofurane or a mixture thereof. Preferably the solvent is water or an aqueous solution. Preferably the reaction mixture is an aqueous solution.

The photo irradiation may be provided by a UV lamp or other light source. Preferably UV irradiation refers to radiation in the range of 350-420 nm. This may be achieved by using a 1000 watt UV lamp.

The monomer may be any monomer capable of forming a conjugated polymer under the conditions described. The monomer may be a phenylic or a heterocyclic aromatic monomer, such as pyrrole, furan, thiophene and their

substituted derivatives. The monomer may be thiophene and/or a derivative thereof. Reference herein to a polymer is intended to include what are more commonly referred to as oligomers.

A derivative of thiophene (Th) may be any 3-substituted thiophene molecule with the formula

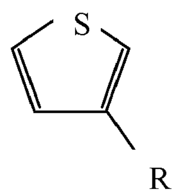

wherein R may be selected from the group comprising a hydrogen, an aliphatic group, alcohol group, amino group, alkoxy group, acryl group, meth(acryl) group, carboxyl group, ester group, (poly)ester group, ether group or (poly)ether group.

An aliphatic group may comprise a C1 to C12, preferably a C1 to C8, preferably a C1 to C6, more preferably a C1 to C4 alkane or alkene. The aliphatic group may be straight or branched chain, and substituted or unsubstituted.

An alcohol group may comprise a C1 to C12 alcohol, preferably a C1 to C8 alcohol, preferably a C1 to C6 alcohol, more preferably a C1 to C4 alcohol.

An amino group preferably comprises $NR_1R_2$, wherein $R_1$ and $R_2$ are independently selected from hydrogen, C1 to C8 alkyl, C1 to C6 alkyl and C 1 to C4 alkyl.

An alkoxy group may comprise a C1 to C6 alkoxy, more preferably a C1 to C4 alkoxy.

A carboxyl group may be -COOR, wherein R is selected from hydrogen, a C1 to C8 alkyl, C1 to C6 alkyl or C1 to C4 alkyl.

An ether group may be $R_A$-O-$R_B$, wherein $R_A$ is a C1 to C8 alkyl, C1 to C6 alkyl or C1 to C4 alkyl; and $R_B$ is a C1 to C8 alkyl, C1 to C6 alkyl or C 1 to C4 alkyl.

Preferably R is methanol and the monomer is 3-thiophene methanol (ThM).

The monomer may comprise a mixture of monomers. The mixture may comprise two or more different monomers selected from thiophene and derivatives thereof.

The nanoparticles produced by the method of the invention may comprise aromatic phenylic or heterocyclic polymers, such as polypyrrole, oligopyrrole, polyfuran, oligofuran, poly(thiophene), oligothiophene and/or a copolymer thereof, and/or derivatives thereof.

Preferably the nanoparticles produced by the method of the invention comprise poly(thiophene) or a polymer of a derivative of thiophene with the above formula, such as poly(3-thiophene methanol), or a copolymer thereof, such as a mixture of oligo(thiophene) and oligo(3-thiophene methanol).

The photosensitising agent may be selected from the group comprising the phenones, benzophenones, peroxides, or any other molecule that decomposes upon UV-irradiation. The photosensitizer is preferably 2-hydroxy-2-methyl-propiophenone (HMPP).

Preferably the amount of photosensitising agent is from about 1 % to about 100 % in respect to the monomer, more preferably in the range about 25 % to about 75 % wherein the % is vol%.

Preferably the photosensitizing agent is immiscible with the solvent and forms droplets.

Exposure of the monomers to the photo irradiation preferably causes polymerisation of the monomer and the production of nanoparticles. In one embodiment, exposure of thiophene monomers and/or derivatives to UV radiation causes the polymerisation of the thiophene monomers and/or derivatives thereof and the production of nanoparticles of poly(thiophene or a derivative thereof) (which includes oligo(thiophene or a derivative thereof).

Preferably the method produces nanoparticles with a low size polydispersity, inferior to 0.5, preferably inferior to 0.2, more preferably inferior to 0.1 and even more preferably equal or inferior to 0.02. The size of the particles produced will be influenced by the conditions used in the method to produce them, for example, the wavelength and length of UV exposure, the type and amount of monomer used etc.

After synthesis the nanoparticles may be lyophilized and/or dried prior to use.

The method of the invention may include adding a crosslinking agent to the reaction mixture. Preferably upon exposure to photo irradiation, such as UV irradiation, the crosslinking agent causes crosslinking to occur within the polymer chains of the nanoparticles.

The crosslinking agent may be one or more of 3-[(2-acryloyloxy)methyl] thiophene (ATh), 3-[(2-methacryloyloxy) methyl] thiophene, 3-[(2-acryloyloxy) ethyl] thiophene, 3-[(2-methacryloyloxy) ethyl] thiophene, 3-[(2-acryloyloxy) propyl] thiophene, 3-[(2-methacryloyloxy) propyl] thiophene, vinylmethyl thiophene, vinylethyl thiophene, vinylpropyl thiophene, epoxymethyl thiophene, epoxyethyl thiophene, epoxypropyl thiophene, etc.

A crosslinker may be any molecule that disposes of one thiophene ring able to copolymerize with the thiophene monomer and thiophene derivatives, and a second polymerizing group that will polymerize separately, thus resulting in a crosslinked network.

Crosslinking within the nanoparticles may result in nanoparticles with improved thermal stability, and/or with improved resistance to extraction using organic solvents, and/or with improved resistance to aggregation.

Preferably the nanoparticles are thermally stable, preferably they are stable at temperatures up to 350°C.

Preferably the nanoparticles are stable in UV-light, preferably they are stable in wavelengths up to 350 nm.

Preferably the nanoparticles are stable in solution, preferably they do not precipitate and remain dispersed in solution, or are easily dispersed upon shaking after long term storage (of 1 year or more).

Preferably the nanoparticles are substantially monodisperse, preferably the polydispersity is inferior to 0.5, more preferably the nanoparticles have a polydispersity 0.002, and even 0.001.

Preferably the nanoparticles are biocompatible.

Preferably the reaction mixture used in the method of the invention is homogenised prior to exposure to photo irradiation.

The reaction mixture may be exposed to photo irradiation, such as UV radiation, for at least 2, 4, 6, 8, 10, 15, 20 or more hours.

Preferably nanoparticles of poly(thiophene) or a derivative thereof produced by the method of the invention are produced in a more environmentally friendly manner than known methods used to produce such nanoparticles. Preferably the method of the invention produces nanoparticles with a low size polydispersity in contrast to known methods.

The method of the present invention is preferably a one step process.

Preferably the method of the invention does not require a metal-containing reagent, such as $Fe^{3+}$ salts or potassium dichromate, as a catalyst. Preferably the method of the invention does not require an organic solvent, which are usually chlorinated. By avoiding a metal-containing reagent and/or an organic solvent the resulting nanoparticles are less toxic, this is particularly important if the nanoparticles are to be used in a composite for use as a biomaterial.

Preferably the nanoparticles produced by the method of the invention do not contain metal or a metallic residue.

Preferably the nanoparticles produced by the method of the invention are biocompatible.

According to a further aspect, the invention provides a nanoparticle produced by the method of the invention.

According to another aspect, the invention provides a nanoparticle obtainable by the method of the invention.

According to yet another aspect, the invention provides a composite comprising nanoparticles of a conjugated polymer.

Preferably the nanoparticles in the composite are made, or are obtainable, by a method of the invention. The method may comprise providing a reaction mixture comprising a monomer, a photosensitising agent and a solvent, and exposing the reaction mixture to photo irradiation to form nanoparticles of a conjugated polymer.

In a preferred embodiment, the conjugated polymer is poly(thiophene) or a derivative thereof or a copolymer of thiophene and a derivative thereof. The derivative of poly(thiophene) may be poly(thiophene methanol).

Preferably the nanoparticles in a composite of the invention are particles wherein at least one dimension, such as the radius, is equal to or lower than about 1000 nm, preferably is equal or lower than about 250 nm and even more preferably is equal or lower than about 100 nm.

Preferably the nanoparticles are substantially spherical and may be referred to as nanospheres, that is, they have a radius of less than about 1000 nm.

Preferably the nanoparticles are stable. That is, they are preferably thermally stable and/or stable on exposure to UV-light and/or are stable in solution.

Preferably the nanoparticles in a composite of the invention do not comprise any metal or metallic residue.

Preferably the nanoparticles in a composite of the invention are substantially all the same size. Preferably the nanoparticles differ by less than about 25%, 20%, 15%, 10% or less in volume. The presence of significantly larger particles would affect the absorption properties of the material. Nanoparticles significantly larger than 100 nm may increase opacification as a result of light scattering.

Preferably the composite with nanoparticles is a nanocomposite, that is, is a multiphase solid material wherein one of the phases has one, two or three dimensions of less than about 1000 nm, preferably less than about 250 nm and even more preferably less than about 100 nm.

Preferably the composite containing the nanoparticles is biocompatible. This may be determined by cytotoxicity and/or cell adhesion studies.

Preferably the nanometric size of the nanoparticles means that they can be eliminated from the body if needed and thus are biodegradable. The nanometric size may also improve the optical properties of the composite; nanoparticles below 100 nm may be transparent.

Preferably the composite comprises a chemically or physically crosslinked polymer that may or may not absorb water. Most preferably the composite is chemically crosslinked and stable upon dissolution. Even more preferably, the composite is a chemically crosslinked polymer capable of absorbing water, thus a hydrogel. A hydrogel may be a network

of polymer chains that are substantially water-insoluble.

Preferably if the composite is a hydrogel and the nanoparticles are easily dispersed in the monomers and/or monomer mixtures used to form the hydrogel.

The hydrogel may be a hydrophilic acrylic hydrogel. Such hydrogels may be used in intra ocular lens implants.

Preferably the hydrogel comprises poly(2-hydroxyethyl methacrylate-co-methyl methacrylate) [poly(HEMA-co-MMA)] and is formed from 2-hydroxyethyl methacrylate (HEMA), methyl methacrylate (MMA) and the cross-linking agent ethylene glycol dimethacrylate (EGDMA)

Preferably a composite according to the invention prevents/reduces the transmission of UV light (up to about 350 nm) when compared to the same material without the nanoparticles. Preferably the reduction is proportional to the amount of nanoparticles incorporated. Most preferably such a composite is capable of absorbing at least about 10% up to about 70 % more of the UV light at 350 nm compared to the neat host polymer (hydrogel, for instance), that is, without nanoparticles, Preferably the composite comprises 2.5 mg nanoparticles made by the method of the invention to every 1 mL of the initial monomer mixture used to prepare the polymer host material.

Preferably a composite according to the invention prevents/reduces the transmission of blue light (350 - 450 nm) when compared to the same material without the nanoparticles. Most preferably a composite according to the invention is capable of absorbing at least about 5 % up to about 30 % more of the blue light at 450 nm compared to the neat host polymer (hydrogel, for instance) when 2.5 mg nanoparticles according to the invention are added to 1 mL of the initial monomer mixture for the preparation of the polymer host material. The composite of the invention containing the nanoparticles may be used in the production of IOL implants which reduce the risk of damage to underlying retinal epithelial cells caused by exposure of the retinal cells to UV and/or blue light.

Preferably, in a composite of the invention with reduced UV transmission properties, the amount of nanoparticles per volume of initial monomer mixture for the preparation of the polymer host material is between about 0.1 and about 10 g/L, more preferably between about 0.5 and about 2.5 g/L.

Preferably if the composite is a hydrogel the inclusion of the particles in the hydrogel has little or no significant effect on the surface properties of the hydrogel. For example, causes no significant increase the attachment of cells to the hydrogel surface.

The composite of the invention may be used as an optical material, for example as a biomaterial in an IOL implant, and/or in solar cells, and/or in photovoltaic cells.

The composite of the invention may be used in an implant, for example in an IOL implant or a dental implant.

The composite of the invention may be used as a biomaterial in a range of contexts, for example in materials, products or substances which are for use in contact with biological samples, tissues, fluids, cells, cell components, etc either *ex vivo, in vivo* or *in vitro.*

According to another aspect the invention provides a method of producing a hydrogel comprising (i) dispersing nanoparticles of a conjugated polymer in a monomer mixture, and (ii) polymerising the monomer around the particles to form a hydrogel containing the nanoparticles. Preferably the nanoparticles are produced by the method of the invention.

Preferably the nanoparticles are nanoparticles of poly(thiophene), or a derivative thereof, such as poly(thiophene methanol), or a copolymer thereof.

Preferably the monomer is a mixture of hydroxyethyl methacrylate (HEMA) and methyl methacrylate (MMA), which polymerises to form poly(HEMA-co-MMA). The crosslinking agent EGDMA may be included in the monomer mix and the polymerisation may be catalysed by ATh.

According to another aspect the invention provides an intraocular lens implant comprising a hydrogel containing nanoparticles of a conjugated polymer.

Preferably the conjugated polymer particles are as described with respect to the first aspect of the invention. Preferably the conjugated polymer particles are made according to the method of the first aspect of the invention.

The particles and/or hydrogel may be as described above.

According to another aspect the invention provides a composite comprising nanoparticles produced by any method of the invention, wherein the composite displays reduced transmission of UV light and/or blue light compared to the composite without nanoparticles.

The composite may be a hydrogel. The hydrogel may be as described herein. The nanoparticles may be as described herein.

It will be appreciated that, where appropriate, all optional or preferable features applicable to one aspect of the invention can be used in any combination, and in any number. Moreover, they can also be used with any of the other aspects of the invention in any combination and in any number. This includes, but is not limited to, the dependent claims from any claim being used as dependent claims for any other claim in the claims of this application.

Embodiments and examples of the present invention will now be described, by way of example only, with reference to the following figures.

**Figure 1 -** shows TEM images of the nanoparticles listed in Table 1. The numbering of the micrographs is the same

as in Table 1.

**Figure 2 -** shows a schematic representation of the formation of nanoparticles of oligo(Th), oligo(ThM) or their co-oligomers in water.

**Figure 3 -** shows the IR spectra of (I) ThM and (II) parent nanoparticles (entry 6, Table 1).

**Figure 4 -** illustrates the UV-Vis spectra of: (A) aqueous solution of Th and HMPP (entry 5, Table 1) before and after UV-irradiation; (B) aqueous solution of ThM and HMPP (entry 6, Table 1) before and after UV-irradiation; (C) nanoparticles listed in Table 1 entries 2 & 4-6.

**Figure 5 -** shows the fluorescence emission spectra of: (A) aqueous solution of Th and HMPP (entry 5, Table 1) before and after UV-irradiation; (B) aqueous solution of ThM and HMPP (entry 6, Table 1) before and after UV-irradiation; (C) nanoparticles listed in Table 1, entries 2, 5 & 6.

**Figure 6 -** shows TEM images of the oligo(ThM) nanoparticles listed in Table 1, entry 6, and redispersed in THF and HEMA after drying.

**Figure 7 -** shows the chemical structure of the cross-linker 3-[(2)acryloyloxy)methyl] thiophene (ATh).

**Figure 8 -** shows TEM images of the nanoparticles prepared under the conditions reported in Table 2. The numbering of the micrographs is the same as in Table 2.

**Figure 9 -** shows the IR spectra of (I) uncross-linked (entry 6, Table 1) and (II) ATh-cross-linked (entry 3a, Table 2) oligo(ThM) nanoparticles.

**Figure 10 -** shows the overlaid UV-Vis spectra of the aqueous dispersions of A) oligo(Th) nanoparticles and B) oligo(ThM) nanoparticles.

**Figure 11 -** shows the effect of the cross-linking of the oligo(ThM) nanoparticles by ATh on the fluorescence emission spectrum.

**Figure 12 -** shows TEM images of the ATh-stabilized oligo(ThM) nanoparticles listed in Table 2 (entry 3a) after redispersion in THF and HEMA.

**Figure 13 -** shows the TGA of the non-stabilized and ATh-stabilized oligo(ThM) nanoparticles.

**Figure 14 -** shows the Raman spectrum of ATh-stabilized poly(ThM) nanoparticles (entry 3a, Table 2) after TGA up to 950˚C, at a heating rate of 10˚C/min.

**Figure 15 -** shows the TEM image of the TGA residue (up to 950˚C) of the oligo(ThM) nanoparticles listed in Table 2, entry 3a.

**Figure 16 -** shows the microscopic observation of the cell cultures after « Extraction test » at 2.5 mg/mL concentration.

**Figure 17 -** shows the spectroscopically determined percentage of living cells after « Extraction test » at 2.5 mg/mL concentration.

**Figure 18 -** shows the microscopic observation of the cell cultures after « Direct contact test » at 30 mg/mL concentration.

**Figure 19 -** shows the spectroscopically determined percentage of living cells after « Direct contact test » at 30 mg/mL concentration.

**Figure 20 -** shows voltamperometric graphs for the samples from Table 3. Sample 1: same as entry 5 (Table 1). Sample 2: similar to entry 4 (Table 1) but at inverted Th/ThM vol. ratio of 2/1 instead of 1/2.

**Figure 21 -** shows a TEM image of the nanoparticles listed in Table 4, entry 6 used for the preparation of the hydrogel

nanocomposite;

**Figure 22 -** illustrates schematically the stabilization of poly(Th-co/or-ThM) nanoparticles within the acrylics monomers precursor for the poly(HEMA-co-MMA) hydrogel;

**Figure 23 -** is a comparison of 1 mm thick poly(HEMA-co-MMA) hydrogel nanocomposites listed in Table 5 with a commercially available **UV-** and blue-light blocking IOL.

**Figures 24A to 24D -** show the UV-vis spectra of the hydrogel nanocomposites listed in Table 5;

**Figure 25 -** shows the UV-vis spectra of the natural human lens crystalline at different ages;

**Figure 26 -** shows the TEM observation of the hydrogel nanocomposites listed in Table 5;

**Figures 27A to 27C -** show DSC curves for the pre-dried nanocomposites listed in Table 5;

**Figures 28A to 28F -** show the dynamic mechanical properties of the hydrogel nanocomposites listed in Table 5;

**Figures 29A to 29C -** show the results of thermogravimetric analysis of the pre-dried nanocomposites listed in Table 5;

**Figure 30 -** shows optical micrographs of LECs adhering to the surface of the hydrogel nanocomposites listed in Table 5, together with a histogram indicating the surface occupied by LECs.

## Examples

### Materials

Thiophene (Th, 99+%), 3-thiophene methanol (ThM, 98%), 2-hydroxy-2-methyl-propiophenone (HMPP, 97%), 2-hydroxyethyl methacrylate (HEMA), methyl methacrylate (MMA) and ethylene glycol dimethacrylate (EGDMA) were purchased from Aldrich, stored in the dark at -20˚C and used without further purification. Azo-bisisobutyronitrile (AIBN, Fluka) was also stored in the dark at -20˚C and used as received. 3-[(2-acryloyloxy)methyl] thiophene (ATh) was synthesized as described below. Milli-Q water (18.2 MΩ) was obtained by a Milli-Q system from Millipore. Acryloyl chloride (AC, Aldrich) and triethylamine (TEA, Aldrich) were dried on $CaH_2$ and used after distillation. All other solvents (tetrahydrofurane (THF), toluene and methanol) were of analytical grade. Conventional yellow lenses (Yellow Flex) from PhysIOL SA, Belgium, were used as reference lenses.

### Synthesis of 3-[(2-acryloyloxy)methyl] thiophene (ATh)

ATh was synthesized according to a slightly modified recipe reported elsewhere (Kock et al. Synth. Metals. 1996, 79, 215-218; Guner et el. Europ. Polym. J. 2004, 40, 1799-1806). Briefly, 1.68 mL (1 mol eq) of ThM, 3.23 mL (1.3 mol eq) of freshly distilled TEA and 20 mL dry THF were added under argon atmosphere to a pre-conditioned double-necked flask equipped with a magnetic stirring-bar and a dropping funnel. The mixture was cooled down to 0˚C in an ice/water bath. A 10 mL solution of 1.89 mL (1.3 mol eq) of freshly distilled AC in dry THF was then added dropwise. The mixture was allowed to warm up slowly to room temperature and to react for 20 h. The ammonium salt precipitate was filtered out. The filtrate was concentrated with a rotary evaporator, and the residue was dissolved in 200 mL diethyl ether. This solution was washed with 80 mL saturated aqueous solution of $NaHCO_3$, then dried over $MgSO_4$, followed by the elimination of the solvent. The oily residue was identified as ATh by NMR and collected with a 62.6% yield. It was used without further purification.
$^1H$ NMR ($CDCl_3$) δ= 6.01 and δ = 6.57 (C**H**$_2$=CH); δ = 6.32 (CH$_2$=C**H**); δ = 5.36 (C**H**$_2$-OCO); δ = 7.46 (C**H**-S-C**H**); δ= 7.26 (C**H**=CH-S, overlapped with the peak for the solvent).

### Characterization

The average radius ($R_{DLS}$) and particle size distribution or polydispersity index ($PDI_{DLS}$) of the nanoparticles were analyzed in water by Dynamic Light Scattering (DLS). Malvern CGS-3 equipped with a He-Ne laser (633 nm) was used. The scattering cell was immersed in filtered toluene and the temperature was maintained at 25˚C. Data were recorded at a scattering angle of 90˚. PDI was defined as the $\mu_2/\Gamma_1^2$ ratio, where $\mu_2$ is the second cumulant and $\Gamma_1$ is

the first cumulant. The DLS data were also analyzed by the CONTIN routine, a method based on constrained inverse Laplace transformation of the data and which gives access to a size distribution histogram for the particles.

Fluorescence emission spectra of the nanoparticles in the aqueous reaction mixture were collected on a Perkin Elmer LS50B spectrofluorimeter at 350 nm excitation wavelength. UV-Vis spectra were recorded with a HP 8453 UV-visible spectrophotometer (Hewlett-Packard, Waldbronn, Germany) fitted with a 1024- element diode-array. Infrared spectra (IR) of dried nanoparticles were collected after 8 scans on a Perkin Elmer 1600 equipment with a 4 $cm^{-1}$ resolution. Raman spectra were recorded with a LabRam spectrometer (Jobin-Yvon) equipped with an Olympus confocal microscope (100x objective lens, 300 $\mu$m confocal pinhole) and a Peltier air-cooled, back illuminated, deep depletion 1024 × 128 CCD detector. The exciting wavelength was 785 nm delivered by a diode laser (Torsana StarBright 785 XM; power of 4 mW). The integration time was fixed at 40 s. The particles to be analyzed were selected by viewing the sample with the CCD camera attached to the microscope. The baseline was corrected and subtracted from the recorded spectra, by using a polynomial regression model. A Savitsky-Golay smoothing was also performed.

Transmission Electron Microscopy (TEM) was used to observe either nanoparticles or microtomed slices (- 80 nm thick, RuO staining) of nanocomposites. The nanoparticles were deposited on Formvar- or carbon- coated copper grids by casting a drop of a suspension in water or in an organic solvent, followed by slow solvent evaporation. A Philips CM 100 TEM equipped with a Gatan 673 CCD camera connected to a computer with the Kontron KS 10 software was used. Differential Scanning Calorimetry (DSC) and Thermogravimetric Analysis (TGA) were performed on samples dried at 50°C under reduced pressure overnight. TGA was carried out with a TA Q500 apparatus at a heating rate of 10°C/min up to 700 or 950°C. DSC was performed with a TA DSC Q100 apparatus, according to the following thermal treatment: (i) cooling to -60°C, (ii) isotherm at -60°C for 5 min, (iii) heating at 15°C/min until 200°C, (iv) isotherm at 200°C for 5 min, (v) fast cooling down to -60°C, (vi) isotherm at -60°C for 5 min, (vii) heating at 15°C/min up to 200°C. The second run of heating was systematically considered.

One mm thick discs of the nanocomposites (8 mm in diameter) were analyzed by Dynamic Mechanical Analysis (DMA, Rheometric Scientific - ARES) at 20°C, with a frequency of 99.903 rad/sec and an initial strain and a final strain of 2.02% and 10.0% respectively. The composites were first equilibrated in a pot filled with water for at least 48h at room temperature. The non-absorbed water which remained on the surface of each sample was gently eliminated by filter paper, since it could impede the good contact between the surface of the sample and the surface of the analytical plate.

Water uptake (W%) was determined according to the equation:

$$W\% = [(m_{hydrated} - m_{dry})/\ m_{hydrated}]*100$$

where $m_{hydrated}$ is the weight of the sample in the fully hydrated state (swelling for at least 48 h at room temperature in a water filled well-closed container ; non-absorbed water was removed from the surface with a filter paper just before weighting) and $m_{dry}$ is the weight of the same sample dried for 48 h at 50°C under reduced pressure.

For the measurement of conductivity, poly(Th-co/or-ThM) nanoparticles (10 mg) were dispersed in 4mL $5x10^{-2}$M tetrabutyl ammonium perchlorate solution in dry acetonitrile. A home-made electrochemical conductometer was used for the measurements.

The cell culture line L929 was used for estimating the cytotoxicity of the nanoparticles of poly(Th-co-ThM), Th/ThM of 1/2 (sample 1) and 2/1 (sample 2) ratio and the eventual compound extracted from the nanoparticles. Suspensions of 2500 cells/well were incubated in 96 well Falcon box at 37°C, 5% $CO_2$ in 100 $\mu$L culture medium (CM, 87% Dulbecco's Modified Eagle's Medium (Cambrex); 10% bovine foetal serum (Gibco) and 1% of antibiotics (penicillin/streptomycin 10 000U/10 000$\mu$g/ml); 1% Glutamine; 1% HEPES). When 10% confluence was achieved after approximately 1 day, the CM was substituted by 100 $\mu$L of the respective test dispersion (containing the nanoparticles, "Direct contact test") or test solution (containing the extracts from the nanoparticles, "Extraction test"). The cells were then incubated at 37°C and 5% $CO_2$ for another 72 h, which was determined to be enough for 80-90% confluence of calibration culture. 20 $\mu$L of tetrazolium salt (MTS) (Kit "The CellTiter 96® Aqueous Non-Radioactive Cell Proliferation Assay") was then added tp each well, this was used as a dye for colorimetric determination of the metabiolic activity of the cell culture. MTS is reduced to formazan derivative by the dehydrogenases of the living cells. The MTS was left to react with the cells for 2 h at 37°C and 5% $CO_2$. The absorption of the CM was then determined at 490 nm, and is proportional to the amount of formazan, which is proportional to the number of living cells.

Nanoparticle dispersions were prepared for the "Direct contact test" in dimethylsulfoxide (DMSO) at concentration of 300 mg/mL. The dispersions were then diluted in CM until a concentration of 30 mg/mL was obtained. It was then used for the test. For the "Extraction test" (the cells are not in direct physical contact with the nanoparticles but with an extract of the particles), the nanoparticles were incubated in CM at concentration of 2.5 mg/mL for 12 days at 37°C. The dispersions were regularly agitated. Finally, they were centrifuged for 10 min at 15 000 G and the supernatant was filtered through a 0.22 $\mu$m filter. 100 $\mu$L/ well (96 well Falcon box) of the filterd supernatant was used for the tests.

Lens epithelial cells (LECs) were isolated from the lens crystalline capsule of pig eyes (Pietrain-Landrace pig; from the "Abattoir communal de Liège") and cultured as reported by Dumaine, L. (Thesis: Correlation entre les proprietes physico-chimiques de polymers permettant de moduler la proliferation cellulaire et celles permettant de favoriser le glissement de lentilles intraoculaires. Ecole Centrale de Lyon. 2004, 79-85). Cells in $4^{th}$ to $10^{th}$ passage were used for testing.

The LECs adhesion test (*in vitro* test) was similar to the one reported by Bozukova et al. Biomacromolecules 8, 2007, 2379-2387. The samples were pre-conditioned in a SERAG BSS® physiological solution (pH 7.3) for 2 days and cut as disks with a 6 mm diameter, thus fitting the test wells. They were washed and sterilized in physiological solution at 1.5 bar, 120˚C for 21 min. They were then fixed on the bottom of a 96 well-plate and conditioned with the culture medium (CM, 87% Dulbecco's Modified Eagle's Medium (Cambrex); 10% bovine foetal serum (Gibco) and 1% of antibiotics (penicillin/streptomycin 10 000 U/10 000 μg/ml); 1% Glutamine; 1% HEPES) in an incubator for 2 h. After removal of the conditioning CM, a suspension of 7500 cells/ 200 μL was added to each well. After 3 days of contact between hydrogel samples and LECs suspension in the incubator, the culture medium was removed, followed by a careful washing with phosphate buffer saline (PBS) in order to eliminate the non-adhering and dead cells. The cells were fixed with 4% paraformaldehyde at 4˚C for 2 h, washed three times with PBS and kept in PBS at 6˚C until microscopic observation.

The sample surface was observed with an optical microscope Zeiss, Axioplan, equipped with Epiplan-NEOFLUAR objectives (5x magnification) and a CCD camera (ICD-42E model). The images were visualized with the XnView software. Each sample was analyzed in triplicate. The surface area occupied by the cells was determined by a software Dpx View Pro Image management.

**Example 1: Preparation and analysis of non-stabilized well-defined nanoparticles of oligo(Th), oligo(ThM) or their co-oligomers**

Predetermined quantities of Th, ThM and HMPP were mixed and then homogenized under vigorous stirring. They were then added to a reaction tube equipped with a magnetic stirring-bar and a predetermined amount of solvent, i.e. Milli-Q water or toluene. The reaction mixture was maintained under stirring for 30 min and then UV-irradiated with a UV-lamp (1000 watt; 350-420 nm) for 20 h, either directly or after a preliminarily de-oxygenation by argon bubbling. The nanoparticles were analyzed in the reaction mixture. Part of them were then collected by lyophilization, followed by drying under reduced pressure overnight at 50˚C before analysis. The exact quantities of all reagents are listed in Tables 1 and 2.

HMPP is immiscible with water, in contrast to Th that forms an azeotropic mixture with water, whereas all of HMPP, Th and ThM are miscible with organic solvents, including toluene. Because of these differences in the solubility of the monomers and sensitizer (HMPP) in water and toluene, these two types of solvents might have an effect on the formation of nanoparticles. Examples are first presented in water (entries 2-7, Table 1) while a comparative example in toluene (entry 1, table 1) follow these.

A 2/1 (v/v) mixture of Th and ThM was polymerized in water (entry 2, Table 1). In all the experiments the one-phase mixture of HMPP and monomers was added to the solvent. The reaction medium was maintained under stirring for 30 min before being UV-irradiated for 20 h. The occurrence of a photo reaction was visible to the naked eye. The colourless mixture turned light yellow as a dispersion of nanoparticles was formed.

In a second series of experiments, the volume ratio of the Th and ThM comonomers in water was changed, such that three compositions could be compared (entries 2 to 4, Table 1). Moreover, each comonomer was photopolymerized separately (entries 5 and 6, Table 1). Fig. 1 (entries 2 to 6) demonstrates that well-defined spherical nanoparticles with narrow size distribution are formed in water, whatever the monomer and the comonomer feed composition. It was noted that nanoparticles of oligo(Th) were at least two times smaller ($R_{DLS}$ = 49 nm) than the oligo(ThM) counterparts ($R_{DLS}$ = 109 nm). Consistently, the average radius of the co-oligomer nanoparticles decreases whenever the Th content is increased.

In an additional experiment, the monomer/HMPP volume ratio was changed by a two-fold increase of the volume of HMPP with respect to that of ThM, the latter was kept unchanged (entry 7, Table 1). Smaller nanoparticles (micrograph 7, Fig. 1) with a broader size-distribution were found for the mixture with a higher amount of HMPP, possibly as the consequence of the faster polymerization, promoted by the HMPP content on the nanoparticles formation. Therefore, the 2/1 (v/v) monomer/ HMPP ratio was selected in the next experiments.

**Table 1:** Experimental conditions for the preparation of nanoparticles of oligo(Th) and derivatives, together with particle size data.

| Entry | Solvent 10 mL | Monomer 10 μL | HMPP μL | $R_{DLS}$ nm | $PDI_{DLS}$ | $R_{TEM}$ nm |
|---|---|---|---|---|---|---|
| 1 | toluene | Th/ThM, 2/1 (v/v) | 5 | 362 | 0.21 | ≈ 250 |
| 2 | water | Th/ThM, 2/1 (v/v) | 5 | 64 | 0.1 | ≈ 50 |
| 3 | water | Th/ThM, 1/1 (v/v) | 5 | 78 | 0.09 | ≈ 60 |
| 4 | water | Th/ThM, 1/2 (v/v) | 5 | 83 | 0.13 | ≈ 70 |
| 5 | water | Th | 5 | 49 | 0.06 | ≈ 35 |
| 6 | water | ThM | 5 | 109 | 0.02 | ≈ 90 |
| 7 | water | ThM | 10 | 90 | 0.27 | ≈ 55 |

It is not surprising that the size of the nanoparticles observed by TEM (Fig. 1) is systematically smaller, compared to DLS data (Table 1). Indeed, although the nanoparticles were analyzed by DLS in solution, they were dried prior to observation by TEM. The elimination of water and residual monomer, the monomer conversion being estimated at approximately 50 wt%, was responsible for the observed contraction of the particles.

Upon lyophilization the nanoparticles were collected as dark brown to black powder, which is typical for the oxidized oligothiophene and derivatives. Evidence of the photo-co-polymerization of thiophene and derivatives was searched by comparative spectroscopic analyses of the monomers (Th and ThM) and the nanoparticles collected after UV-irradiation.

The IR spectrum of ThM shows the symmetric and antisymmetric stretching vibrations of the thienyl ring at 1335 $cm^{-1}$ and 1416 $cm^{-1}$, respectively (Fig. 3 - I). These bonds are shifted upon UV- irradiation towards wavelengths characteristic of α-coupled thiophene units (Fig. 3 - II) which testifies that the polymerization occurred. Moreover, the absorption at 1155 $cm^{-1}$ by the C-S bond of the monomeric thienyl ring is shifted towards smaller wave-numbers after UV-irradiation, as a possible consequence of the conjugation in the polymer chains. The absorption at 2875 $cm^{-1}$ must be assigned corresponding to the C-H stretching vibration of the methylene group of both ThM and ThM monomeric units in the chains, without distinction. The hydroxyl group of the ThM is at the origin of the absorption at 3300 $cm^{-1}$. The more convincing signature of polymerization has to be found at 1688 $cm^{-1}$, which is typical of the aromatic C=C bending in conjugated chains.

UV-Vis spectra are shown in Fig. 4, for Th and ThM, in aqueous solution with HMPP, before and after UV-irradiation (entries 5 and 6, Table 1). An absorption maximum was observed at 250-300 nm for the nonirradiated solutions (Fig. 4, A and B). This maximum did not completely disappear upon exposure to UV, because the monomer conversion was systematically limited to - 50%. Nevertheless, an extra, although flat, maximum was observed at higher wavelengths as a result of UV-irradiation for 20 h and thus of partial polymerization. This maximum is higher when ThM is compared to Th and, accordingly, it decreases with ThM content in case of the Th/ThM mixture (Fig. 4 - C).

The fluorescence emission spectra of the reactive aqueous dispersions were also recorded before and after UV- irradiation. An emission maximum of low intensity was observed at 425 nm with a long tail up to 600 nm for the aqueous solutions of each monomer (Th and ThM) and HMPP (Fig. 5, A and B). Although this maximum grew in intensity upon UV- irradiation, only a small shift towards higher wavelengths was observed, which suggests the formation of oligomers rather than long conjugated chains. Nevertheless, this chain-length was enough for well-defined solid nanoparticles to be formed. The fluorescence emission was more important for ThM than for Th (Fig. 5 - C).

Interestingly, the aqueous solutions of each monomer added with HMPP could be stored in the dark, for at least 6 months without any modification. In contrast, they turned light yellow upon exposure to sunlight for 2 weeks, and a very low amount of solid nanoparticles was observed by TEM. This observation supports the suggested UV-photo-induction of the polymerization process. No polymerization occurred and nanoparticles were not formed whenever the aqueous solutions of monomers were UV-irradiated in the absence of HMPP, which confirms the role of photo-sensitizer of this propiophenone derivative.

The nanoparticles collected have a strong tendency to agglomerate when the solvent is evacuated and to resist redispersion afterwards. For instance, oligo(ThM) nanoparticles (entry 6, Table 1) led to a black powder upon lyophilization, which could not be redispersed in water. However partial dispersion was observed in THF and HEMA, respectively,

after 1 h of stirring at original concentrations of 1 mg/ mL. The persistent aggregates were filtered out, and particles of the brown-coloured dispersions were observed by TEM on carbon-coated copper grid (Fig. 6). The average radius, $R_{TEM}$, of the dry particles was 20-25 nm in THF and approximately two times higher in HEMA (- 50 nm), compared to $R_{TEM}$ of ~ 90 nm for the initial nanoparticles. These figures strongly suggest that part of the oligomers were extracted from the particles whose size decreased accordingly.

Comparative example 1:

In order to demonstrate the effect of the solvent, a 2/1 (v/v) mixture of Th and ThM was polymerized in toluene (entry 1, Table 1) under otherwise identical conditions. It was observed that the co-oligomers of Th and ThM formed large particles with a broad size-distribution in toluene (entry 1 in Table 1 and Fig. 1), whereas small spherical particles with low size polydispersity were collected in water (entry 2 in Table 1 and Fig. 1). The origin of this shape contrast might be found in the formation of nano-droplets of HMPP in the aqueous reaction medium, which is not the case in toluene. The monomer molecules that are originally dissolved in these nanodroplets of sensitizer are the first to be polymerized under UV-irradiation, while their consumption is compensated by diffusion of molecules from the aqueous phase into the monomer-depleted nanoparticles. Fig. 2 illustrates the templating role of the HMPP nanodroplets in the formation of oligomer nanoparticles in water.

It must be noted that the HMPP droplets formed in the aqueous phase are in the nanometer to micrometer range and display an extremely high size polydispersity (higher than 0.6) in sharp contrast to the final nanoparticles collected after UV-polymerization. The breaking/coalescence balance of the dispersed phase is thought to change at the expense of the coalescence as the *in situ* polymerization progresses and thus the internal viscosity increases.

The particle formation is completely different in toluene, i.e. in a homogeneous reaction medium. The oligomers that grow in solution rapidly become insoluble, precipitate and aggregate, so leading to poorly defined big-particles. Therefore, water is preferred as the reaction medium to obtain well-defined particles.

**Example 2: Preparation and analysis of stabilized well-defined nanoparticles of oligo(Th), oligo(ThM) and their co-oligomers by cross-linking**

For this purpose, a dual monomer was synthesized, i.e., a compound with (i) a thiophene unit able to copolymerize with Th or ThM, and (ii) an acrylic function prone to radical polymerizion under the experimental conditions used. 3-[(2)acryloyloxy)methyl] thiophene (ATh, Fig. 7) meets these requirements and was synthesized as reported above. The desired quantities of the monomer (Th and ThM), HMPP and ATh were mixed together before being added to the aqueous phase. Because of their intrinsic water-immiscibility, ATh and HMPP formed organic droplets dispersed in water (Fig. 8). Moreover, the partial solubility of the monomers within these droplets is thought to be increased as a result of $\pi$-$\pi$ interactions with ATh. The predicted mechanism of particles formation is basically the same as previously proposed, i.e., copolymerization of ATh and the monomer originally in the droplets, followed by conversion of the monomer that diffuses from the aqueous phase to the droplets, which are more or less rapidly depleted in ATh. Simultaneously to the incorporation of ATh in the conjugated chains the acrylic substituents photopolymerize and trigger the cross-linking of the nanoparticles. So, the structure, i.e., the cross-linking density, of the particles might be heterogeneous to an extent that depends on the partition of the monomer between the aqueous phase and the organic droplets, the reactivity ratios of ATh and the monomer, and the amount of monomer that diffuses into the growing particles and polymerizes *in situ.*

The results of experiments carried out with 3.3 vol% and 33.3 vol% of ATh with respect to the monomer are listed in Table 2. A series of experiments was performed at variable conditions in order to investigate the effect of the monomer composition on the nanoparticles characteristics.

**Table 2:** Experimental conditions for the preparation of ATh- cross-linked nanoparticles of oligo(Th), oligo(ThM) and their co-oligomers. Reactions were conducted in 10 mL water with 5 $\mu$L HMPP.

| Entry | Monomer 10 $\mu$L | ATh $\mu$L | *RDLS* nm | $PDI_{DLS}$ | $R_{TEM}$ nm |
|---|---|---|---|---|---|
| 1a | Th | 0.33 | 43 | 0.02 | 35 |
| 2a | Th/ThM, 1/2 (v/v) | 0.33 | 45 | 0.01 | 35 |
| 3a | ThM | 0.33 | 47 | 0.02 | 40 |
| 4a | Th | 3.33 | 35 | 0.01 | 30 |

(continued)

| Entry | Monomer 10 μL | ATh μL | *RDLS* nm | $PDI_{DLS}$ | $R_{TEM}$ nm |
|---|---|---|---|---|---|
| 5a | Th/ThM, 1/2 (v/v) | 3.33 | 42 | 0.01 | 33-35 |
| 6a | ThM | 3.33 | 45 | 0.1 | 38-40 |

The addition of the lower amount of ATh to the polymerization medium of Th (entry 1a, Table 2) had only a minor effect on the particle size, thus $R_{DLS}$ = 43 nm *vs* 49 nm in the absence of ATh, while $R_{TEM}$ did not change. A small contraction of the wet particles might be the result of cross-linking. The situation was very different when ThM was substituted for Th. In this case the nanoparticles formed in absence of ATh were at least two times larger (109 nm vs 49 nm, Table 1). This effect was completely erased by the addition of ATh ($R_{DLS}$ = 47 nm vs 43 nm for Th, Table 2).

Copolymerization of ATh with a mixture of Th and ThM led to nanoparticles with a size intermediate to that for each of these two monomers (entry 2a, Table 2). ATh had no significant impact on the size polydispersity. The ten-fold increase in ATh in the polymerization medium (entries 4a to 6a; Table 2) decreased the size of the oligoTh nanoparticles by approximately 15%, which might be the consequence of a higher cross-linking density. Surprisingly there was no effect on the size of the oligoThM nanoparticles.

The main spectra that were reported and discussed for the uncross-linked nanoparticles were also recorded for the ATh- cross-linked counterparts. The incorporation of ATh in the chains of oligo(ThM) was assessed by IR spectroscopy, even when only 3.3 vol% was used. Fig. 9 shows the absorption characteristics of the C=O stretching vibration of the acrylate group of ATh at 1758 $cm^{-1}$. Moreover, the absorption typical of the C-H stretching, in the 2780 $cm^{-1}$ to about 3050 $cm^{-1}$ range dominates that one of the hydroxyl substituents of ThM where ATh is added. The contribution of the acrylic component of ATh is a reasonable explanation.

Fig. 10 is a comparison of the UV-Vis spectra of the aqueous dispersions of uncross-linked and ATh-cross-linked nanoparticles, prepared with the same Th (or ThM)/ HMPP composition. The major information is that the absorption by the cross-linked nanoparticles is higher above 350 nm with an extension to higher wavelengths (notably for oligoThM) compared to the non-cross-linked nanoparticles.

The intensity of the emission maximum observed in the 350-600 nm range of the fluorescence emission spectrum of the oligo(ThM) nanoparticles increases dramatically upon cross-linking (Fig. 11). Therefore, the main effect of the ATh would be to increase the ThM conversion (approx. 70%), rather than to increase the conjugated chain length.

In order to confirm that the stability of the nanoparticles towards organic solvents and monomers was improved by cross-linking, oligo(ThM) nanoparticles from entry 3a, Table 2, were dried by lyophilization and then dispersed (1 mg) in 1 mL organic phase (THF and HEMA). Stable dispersion without visible aggregates was immediately (within less than 5 min) obtained, but it was kept under stirring for 1 h in order to provide it the same time of contact between the particles and the organic phase, as in the case of the uncross-linked nanoparticles. The spherical particle morphology was preserved in each solvent (Fig. 12- A and B). Compared to $R_{TEM}$ before redispersion (40 nm), the average particle size decreased by ~15% after dispersion in THF and not at all after dispersion in HEMA. An effective stabilization may be claimed, when compared to a size decrease of ~ 70% and 45% when the uncross-linked nanoparticles were redispersed in THF and HEMA, respectively.

The cross-linking of the oligo(Th) (and derivatives) nanoparticles by even a low amount of ATh (3.3 vol% with respect to the monomer) makes the particles resistant not only to organic solvents but also to aggregation when dispersed in these solvents and stored in water. Stable dispersions in monomers, such as HEMA, are ideal precursors of nanocomposites.

**Example 3: Preparation and analysis of thermally stable nanoparticles and formation of carbon nanoparticles**

The thermal stability of nanoparticles is another important characteristic feature, this was investigated by TGA on a comparative basis for the non-stabilized, uncross-linked and the ATh-cross-link-stabilized oligo(ThM) nanoparticles (entry 6 in Table 1, and entry 3a in Table 2, respectively). Non stabilized uncross-linked nanoparticles were prepared according to Example 1 and cross-link-stabilized nanoparticles were prepared according to Example 2.

A maximum weight loss was observed at 128°C, for the non-stabilized sample (Fig. 13), this most likely results from the elimination of the lower molar mass oligomers. This fraction amounts to approximately 35 wt%. Upon increasing the temperature, longer oligomers were progressively lost as shown by the slowly decreasing slope of the curve until 700°C. A residue of 22 wt% was left at that temperature.

Cross-linking by ATh was beneficial to the thermal stability of the nanoparticles, and the maximum weight loss was shifted upwards (Fig. 13) by 30°C. In addition to the lowest molar mass ThM-oligomers, polyacrylate segments

could contribute to this loss peak that extended up to ~225°C and accounted for 20 wt% of the sample. A slow decomposition was again observed up to 700°C, at which a residue of 39 wt% was noted compared to 22 wt% for the unstabilized particles. At 950°C, the residue decreased down to 29 wt%.

The latter residue was then analyzed by Raman spectroscopy (Fig. 14) and two peaks at 1591 $cm^{-1}$ and 1306 $cm^{-1}$ were observed. According to Qiao et al. (Carbon 2006, 44(1), 187-190), these peaks are the signatures of etches and basal planes in carbon nanoparticles, respectively. The spectrum of the particles before TGA could not be recorded because of a very strong fluorescence.

Part of the TGA residue collected at 950°C was dispersed in ethanol, and a drop of the dispersion was deposited on a carbon-coated TEM grid. After solvent evaporation, spherical particles were observed by TEM (Fig. 15) with $R_{TEM}$ ~ 20 nm, compared to 40 nm for the original ones (Fig. 8, 3a). In conclusion, carbon nanoparticles, were formed by thermolysis.

The herein prepared nanoparticles of oligo(Th) and derivatives are thus potential precursore of carbon nanoparticles by graphitization.

**Example 4: Preparation and analysis of biocompatible nanoparticles**

As the nanoparticles have the potential to be used in biological applications, such as in IOL implants, the cytotoxicity of the nanoparticles was determined. The cytotoxicity of crosslink-stabilized nanoparticles prepared according to Example 2 composed of Th/ThM at volume ratios of 1/2 (sample 1, entry 4 - Table 4) and 2/1 (sample 2, similar to entry 4 - Table 4, but at Th/ThM ratio 1/1 (v/v)) was tested by "Direct contact test" and "Extraction test".

No significant difference was observed in the number of surviving cells from the positive control and the test wells. The slight increase of the absorbance of the test wells culture medium (CM) is probably due to the absorption of the nanoparticles themselves and not to the higher amount of formazan, and higher number of cells, respectively (Fig. 16).

Numerous cells with an aspect similar to that of the positive control well were observed upon microscopic inspection of the culture (Fig. 17), which is in compliance with the non-cytotoxicity of the particles determined spectroscopically. A similar result was obtained by the « Extraction test », for which a concentration of 2.5 mg/mL was used, similar to that incorporated in the poly(HEMA-co-MMA) hydrogel. Again, the percentage of surviving cells varied by ± 5% between the positive control and the test wells, which demonstrates the non-cytotoxicity of the extracts at that concentration (Fig. 18). Upon microscopic observation, the cells seemed to have similar morphology and aspect for the positive control and the test wells (Fig. 19).

**Example 5: Preparation and analysis of conductive nanoparticles**

Stabilized nanoparticles were prepared according to Example 2. Conductivity measurements were performed with 10 mg ATh- stabilized nanoparticles of poly(Th-co/or-ThM) dispersed in 4mL $5x10^{-2}$M tetrabutyl ammonium perchlorate solution of dry acetonitrile (Table 3). The voltamperometric curves are presented in Fig. 20.

**Table 3:** Composition of nanoparticles tested for their conductivity

| Sample | Composition |
|---|---|
| 1 | Same as entry 4 - Table 4 |
| 2 | Similar to entry 4 - Table 4, but at Th/ThM ratio 1/1 (v/v) |

It was observed that whatever the Th/ ThM ratio in the nanoparticles an anodic peak appeared at approx. -0.6 V and a cathodic one at approx. + 1.15 V (Fig. 20). These two peaks speak for the conductive nature of polymer. They are not very strong at these test conditions, probably because only a few particles are in intimate contact with the electrode and may thus contribute to the electroconductivity. However, at optimized conditions, the particles may be useful for the preparation of solar and photovoltaic cells, biosensors, etc.

**Example 6: Preparation and analysis of a hydrogel nanocomposite**

Non stabilized uncross-linked poly(Th-co/or-ThM) nanoparticles were prepared according to Example 1 and cross-link-stabilized nanoparticles were prepared according to Example 2. The experimental data and size characterization features are recorded in Table 4.

Whatever the concentration of the reagents used the nanoparticles produced were systematically spherical with a narrow size-distribution, as shown in Fig. 21. Moreover, the average diameter of the nanoparticles systematically decreased upon both the substitution of Th for ThM and the crosslinking by increasing amounts of ATh (Table 4).

The nanoparticles as described in Table 4 were then used in the production of poly(HEMA-*co*-MMA) hydrogel nanocomposites.

**Table 4:** Experimental data for the preparation of non-stabilized and ATh-stabilized poly(Th-co/or-ThM) nanoparticles.

| Entry | Monomer 10 μL | ATh μL | $R_{DLS}$ nm | $PDI_{DLS}$ | $R_{TEM}$ nm |
|---|---|---|---|---|---|
| 1 | ThM | 0 | 218 | 0.019 | ~ 180 |
| 2 | Th/ ThM, 1/2 (v/v) | 0 | 166 | 0.128 | ~ 140 |
| 3 | Th | 0.33 μL | 86 | 0.0183 | 70 |
| 4 | Th/ThM, 1/2 (v/v) | 0.33 μL | 90 | 0.0143 | 70 |
| 5 | ThM | 0.33 μL | 94 | 0.0209 | 80 |
| 6 | Th | 3.33 μL | 70 | 0.0092 | 60 |
| 7 | Th/ThM, 1/2, (v/v) | 3.33 μL | 84 | 0.0152 | 65-70 |
| 8 | ThM | 3.33 μL | 90 | 0.105 | 75-80 |

The addition under stirring of an aqueous dispersion of nanoparticles (1mg/mL) into the acrylic monomers precursor of the hydrogel, was unsuccessful because of unavoidable phase separation during polymerization. Therefore, the nanoparticles were recovered from the dispersion and dried (cfr Experimental Part), being then easily redispersed in the mixture of comonomers (95 vol% HEMA/ 4.9 vol% MMA/ 0.1 vol% EGDMA) at a content of 2.5 and 10 mg/ mL of comonomers, respectively. ATh (10 μL/ 1 mL monomer) was systematically added to the comonomers, in order to promote interactions between the poly(Th-co/or-ThM) nanoparticles and chemically similar thiophene units incorporated in the hydrogel network. As schematically shown in Fig. 22, the ATh-molecules might be adsorbed onto the nanoparticles surface with their acrylic unsaturation exposed to the copolymerization medium, which foreshadows a mechanism of stabilization of the nanoparticles within the hydrogel poly(HEMA-coMMA) matrix.

**Table 5:** Experimental data for the preparation of poly(HEMA-co-MMA) hydrogel nanocomposites

| Entry | Code Table 4 | Nanoparticles Th/ ThM/ ATh mL | Nanoparticles content mg/ mL comonomers | Water uptake % |
|---|---|---|---|---|
| 1a | Non-filled hydrogels with the basic composition: 95 vol% HEMA/ 4.9 vol% MMA/ 0.1 vol% EGDMA | | | 35 |
| 2a | Entry 1 | 0/ 10/ 0 | 10 | 23 |
| 3a | Entry 2 | 3.33/ 6.67/ 0 | 10 | 19 |
| 4a | Entry 5 | 0/ 10/ 0.33 | 10 | 31 |
| 5a | Entry 3 | 10/ 0/ 0.33 | 2.5 | 36 |
| 6a | Entry 4 | 3.33/ 6.67/ 0.33 | 2.5 | 35 |
| 7a | Entry 5 | 0/ 10/ 0.33 | 2.5 | 35 |
| 8a | Entry 6 | 10/ 0/ 3.33 | 2.5 | 36 |
| 9a | Entry 7 | 3.33/ 6.67/ 3.33 | 2.5 | 35 |
| 10a | Entry 8 | 0/ 10/ 3.33 | 2.5 | 36 |

As a rule, the pre-dried poly(Th-*co*/*or*-ThM) nanoparticles were redispersed quasi-qualitatively within the HEMA/MMA/EGDMA/ATh mixture. It is only when the nanoparticles were not stabilized by effective crosslinking with ATh that a negligible amount of aggregates could be observed. Any aggregation was inhibited and completely transparent dispersions were formed when the nanoparticles were prepared with a higher amount of ATh (entries 8a-10a, Table 5). Two contents of nanoparticles in the comonomer feed were considered (Table 5). When the content of the same nanoparticles was increased from 2.5 mg to 10 mg per mL of comonomers (e.g., entries 4a and 7a, Table 5), the yellow colour turned brown, as illustrated in Fig. 23 for these samples.

The nanoparticles incorporated within the hydrogel were observed by TEM (Fig. 26). A homogeneous distribution was the rule. Due to the thickness of the slices prepared by microtomy and the fact that not all the nanoparticles

are observed with the same TEM focalisation distance, means the average size of the dispersed nanoparticles cannot be determined reliably from the TEM pictures. However, the apparent size is in qualitative agreement with the size reported for the nanoparticles before the hydrogel loading (Table 4).

The preparation technique that consists in dispersing the preformed nanoparticles within the acrylic comonomers precursor of the hydrogel, in the presence of a 3-[(2-acryloyloxy)methyl] thiophene (ATh), leads to a homogenous dispersion of the nanofiller.

**Example 7: Preparation and analysis of a hydrogel nanocomposite as IOL implant**

Recently, Holladay (Eurotimes, September 2007, 24) addressed the question of the possible deleterious impact of the blue-light absorbing IOLs on the vision quality. He wrote that "the lens would not look yellow if it didn't affect colour vision". Therefore, hydrogel nanocomposites listed in Table 5 were compared with a conventional yellow hydrogel IOL (YellowFlex, PhysIOL). Fig. 23 emphasizes a strong decrease in the yellow colour when 2.5 mg of nanoparticles were dispersed in 1 mL of acrylic comonomers (Fig. 23, 5a - 10a), compared to the reference. Although weaker, the same effect holds when the hydrogel was loaded by 10 mg of nanoparticles per mL comonomers (Fig. 23, 2a - 4a).

The UV- and blue-light blocking ability of the poly(HEMA-co-MMA) nanocomposites was established by recording the UV-vis spectra of 1 mm thick samples. Fig. 24-A shows that the unloaded hydrogel (1a) absorbs light at and below 250 nm, whereas the transmission exceeds 89% at 430 nm (in the blue light region) which would be harmful to the eye. The addition of thiophene containing nanoparticles to the hydrogel shifted the absorption towards the UV- and blue- light region (Fig. 24A and D). This effect is more dramatic as the content of nanoparticles in the composition is increased (Fig. 24A, 4a and 7a). Indeed, 36% of light was transmitted at 430 nm when poly(ThM) nanoparticles were used at 10mg of nanoparticles per ml of acrylic monomer (4a in Table 5) compared to 51 % for a four times lower content of nanoparticles. Although beneficial, this effect is not dramatic with respect to the 89% of transmittance observed with the neat hydrogel at the same wavelength (Fig. 24A, 1a).

The UV- and blue- light blocking property of the hydrogel nanocomposites was also sensitive, at least to a moderate extent, to the degree of crosslinking of the nanoparticles. For instance, a ten-fold increase in the amount of crosslinker (ATh) used for the poly(ThM) nanoparticles preparation (1a vs. 7a in Table 5) resulted in a higher transmittance at 430 nm from 51% to 75% at the same nanoparticles content (Fig. 24B and 24C - 7a and 10a).

The nanocomposite comprising poly(ThM) nanoparticles with both the lower content and the lower crosslinking degree (Fig. 24A; 7a) appears to be better at reducing UV and blue light transmission mimicking the commercially available yellow IOL implant (Fig. 24A, Helio25, PhysIOL). As a rule, a change in the Th/ThM volume ratio of the nanoparticles, all the other characteristic features being the same, had only a minor influence on the light-transmitting properties, as shown in Fig. 24B and 24C. Although the hydrogel nanocomposite 3a (Table 5) exhibited the higher UV- and blue- light blocking performance in the series studied (Fig. 24D and 24A), it should be noted that the benefits of the nanocomposite 7a which is stabilized by crosslinking (no aggregation) and used at the lower content (weaker coloration) should be balanced with that of 6a. Most of the hydrogel nanocomposites under consideration in Table 5 not only transmit more than 90 % of visible light but their UV-vis spectrum is also similar to the one of the lens crystalline of a 4 years old child (Fig. 25), which is advantageous to the visual comfort of the patient.

Hydrogel nanocomposites were analyzed by DSC before and after drying, with a special attention paid to the glass transition temperature (Tg). Fig. 27A shows that the dried unfilled hydrogel (1a) has a Tg of 104 ˚C, consistent with the same value reported by Li et al. (Polymer 2007, 48, 3982-3989) for poly(HEMA). The Tg of poly(HEMA-coMMA) was increased by - 10˚C upon loading with 0.25 mg nanoparticles/mL acrylic comonomers, whatever their chemical composition and degree of crosslinking (Figs. 27A (5a), 27B and 27C). The magnitude of this effect should increase with the content of the nanoparticles, which might explain that Tg was no longer detected by DSC when this content was increased four times (Fig. 28A, 2a-4a).

Because hydrophilic acrylic IOL implants are hydrated in use, the herein described nanocomposites were tested as hydrogels and not only in the dried state as was the case for TEM observation and DSC analysis. The swelling of any crosslinked polymer by a ligand results in a loss of modulus and mechanical strength (Meakin et al. J. Mater. Sci.: Mater. Medic. 2003, 14, 783-787; Anseth et al, Biomaterials 1996, 17, 1647-1657). When IOL implants are concerned, a high impact resistance is not required. They just have to resist folding when inserted in the eye. In this work, the hydrogel nanocomposites were analyzed by DMA at 20˚C with a flat-plate geometry and 1 mm thick hydrogel discs (diameter: 8 mm). The major problem faced in these experiments is the slipping of the oscillating plate on the hydrogel surface.

In the strain range investigated in this work, (0.02% to 8%), the unloaded hydrogel behaves as an elastic material, the storage (G') and loss (G") moduli being strain independent, as is the damping factor ($\tan \delta = G''/G'$) (Fig. 28, 1a). This observation is consistent with the analysis of poly(HEMA) hydrogels with the same water content (35 wt%) by Meakin et al. (J. Mater. Sci. Mater. Medic. 2003, 14, 783-787). Typically, the samples with the higher content of nanoparticles (2a and 3a) have a much higher storage modulus, at least at low strains, than all the other nanocomposites

that are 4 times less loaded (Fig. 28A and 28E). This filling effect reinforces the materials resistance to deformation. The sharp decrease in G' and the sharp increase in tan δ with increasing strain, which is observed for the highly loaded samples 2a and 3a (Fig. 28A), is an experimental artefact related to the aforementioned problem of slipping of the oscillating plate on the surface of the nanocomposites with the lower content (19-23 wt% compared to 35 wt% for the other samples).

The nanocomposites with the lower content of nanoparticles can be discriminated on the basis of the crosslinking degree (and not the chemical composition) of the constitutive nanofiller. Indeed, G' and G" are substantially higher when the nanoparticles were prepared with ten times more crosslinker (ATh), as emphasized by the samples 9a and 10a compared to the less crosslinked counterparts 6a and 7a (Fig. 28D and 28E). Another reason is the reinforcing ability of the stiff filler. Once again, a discontinuity is observed in the strain dependence of G' and G" (and an increase in tan δ) for the samples 9a and 10a, which is another signature of the "slipping" problem faced in the DMA measurements.

The glass transition temperature of the dried polyacrylic network increased by - 10°C at the lower content of nanoparticles, which demonstrates that the nanoparticles interact with the ATh co-units of the hydrogel and accordingly have a reinforcing action. The nanofilling also has an impact on the dynamic mechanical properties of the hydrogel. The storage modulus increases with the loading. However, at a constant loading, this modulus is very sensitive to the crosslinking degree of the nanoparticles.

The effect of the nanoparticles on the thermal stability of the polymeric network was also investigated by thermogravimetric analysis. At the higher loading content, whatever the chemical composition of the nanoparticles (samples 2a-4a), Fig. 29A and Table 6 show that the nanofiller has a negative impact on the thermal stability of the matrix. Indeed, the first weight loss is decreased by 35-38°C ($Td^1$), and the second one by 73°C ($Td^2$). The two-step decomposition is typical of the polymeric matrix. It is less affected by the nanofilling when the content of the nanoparticles is decreased (comparison of samples 5a to 10a with samples 2a to 4a Table 6).

According to the data reported in Table 6, the nanoparticles should not perturb significantly the stability of the hydrogel at the sterilization temperature and time used for IOL implants (120°C for 21 min, respectively).

Table 6: Decomposition temperatures of the pre-dried nanocomposites listed in Table 5.

| Entry | $Td^1$(°C) | $Td^2$ (°C) |
|---|---|---|
| 1a | 393 | 496 |
| 2a | 358 | 424 |
| 3a | 356 | 423 |
| 4a | 355 | 423 |
| 5a | 380 | 425 |
| 6a | 394 | 430 |
| 8a | 396 | 427 |
| 10a | 388 | 430 |

As the nanocomposites/hydrogels may be used in IOL implants their biocompatibility is important, in particular their ability to inhibit cell adhesion. A common complication after cataract surgery is Posterior Capsular Opacification (PCO) which occurs due to the adhesion of lens epithelial cells (LECs) to the posterior side of both the lens and the capsular bag. Therefore the ability of LECs to adhere to nanocomposites/hydrogels according to the invention was investigated by an *in vitro* cell adhesion test.

Fig. 30 shows a slight increase in the cell adhesion to the hydrogel surface upon loading with thiophene containing nanoparticles. A possible explanation might be the partial exposure of some of the presumably hydrophobic nanoparticles to the hydrophilic surface, which would create heterogeneities known for enhancing the cell adhesion. However, whatever the nanoparticles, a cell occupation of less than 8% of the surface is estimated and testifies resistance of the nanocomposites to cell adhesion and that the nanocomposites have the potential to resist posterior capsular opacification after cataract surgery.

## Claims

**1.** A method for the production of nanoparticles comprising a conjugated polymer, wherein the method comprises (i)

providing a reaction mixture comprising a monomer, a photosensitising agent and a solvent, and (ii) exposing the reaction mixture to photo irradiation to form nanoparticles of a conjugated polymer.

**2.** The method of claim 1 wherein the solvent is water or an aqueous solution.

**3.** The method of claim 1 or 2 wherein the monomer is thiophene or a derivative thereof, wherein the derivative is of formula (I):

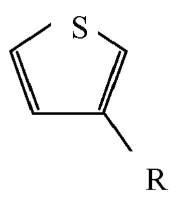

(I)

wherein R may be selected from the group comprising a hydrogen, an aliphatic group, an alcohol group, an amino group, an alkoxy group, an acryl group, a meth(acryl) group, a carboxyl group, an ester group, a (poly)ester group, an ether group or a (poly)ether group.

**4.** The method of any preceding claim further comprising a crosslinking agent.

**5.** The method of any preceding claim wherein the ratio of photosensitizing agent to monomer is in the range 1 % to 100 % wherein the % is vol%.

**6.** The method of any preceding claim wherein the photosensitizing agent is immiscible with the solvent and forms droplets.

**7.** A nanoparticle produced by the method of any of claims 1 to 6.

**8.** A composite comprising nanoparticles of a conjugated polymer.

**9.** The composite of claim 8 wherein the nanoparticles are produced by any of the methods of claim 1 to 6.

**10.** The composite of claim 8 or 9 wherein the nanoparticles comprise poly(thiophene or a derivative thereof), or a copolymer of thiophene and/or derivatives thereof.

**11.** The composite of claim 8, 9 or 10 wherein the composite is a hydrogel comprising nanoparticles of a conjugated polymer.

**12.** The composite of any of claims 8 to 11 wherein the material is biocompatible.

**13.** The composite of any of claims 8 to 12 for use as an optical material.

**14.** A method of producing a hydrogel comprising (i) dispersing nanoparticles produced or obtainable by the method of any of claims 1 to 7, and (ii) polymerising the monomer around the nanoparticles to form a hydrogel containing the nanoparticles.

**15.** The method of claim 14 wherein the monomer is a mixture of hydroxyethyl methacrylate (HEMA) and methyl methacrylate (MMA), which polymerises to form poly(HEMA-co-MMA).

**16.** An intraocular lens implant comprising a hydrogel containing nanoparticles of a conjugated polymer obtained or obtainable by the method of any of claims 1 to 6.

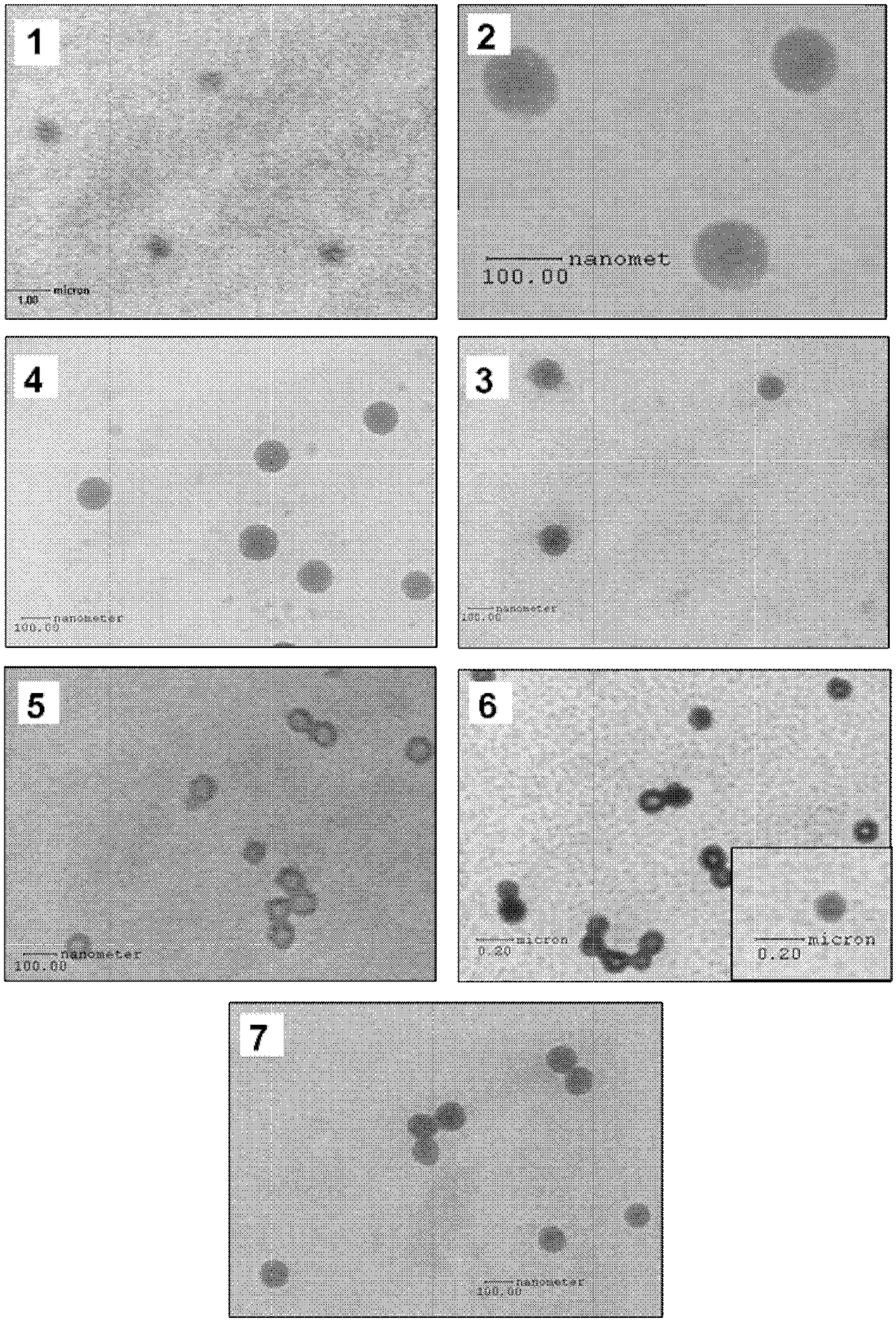
1
1.00 micron
2
100.00 nanomet
4
100.00 nanometer
3
100.00 nanometer
5
100.00 nanometer
6
0.20 micron
0.20 micron
7
100.00 nanometer

Figure 1

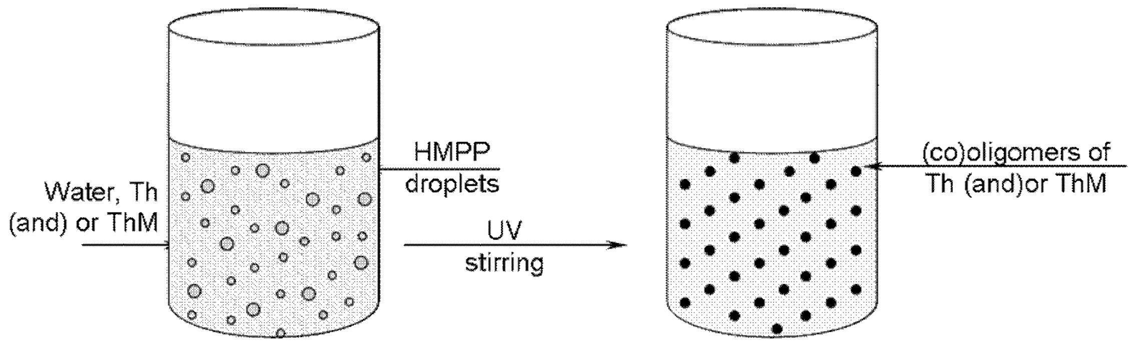


Figure 2

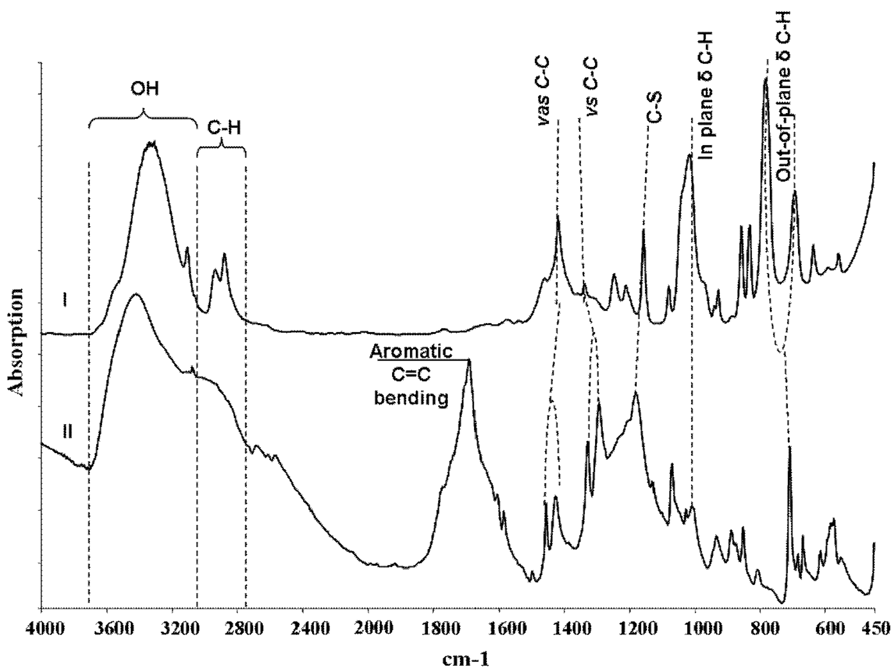


Figure 3

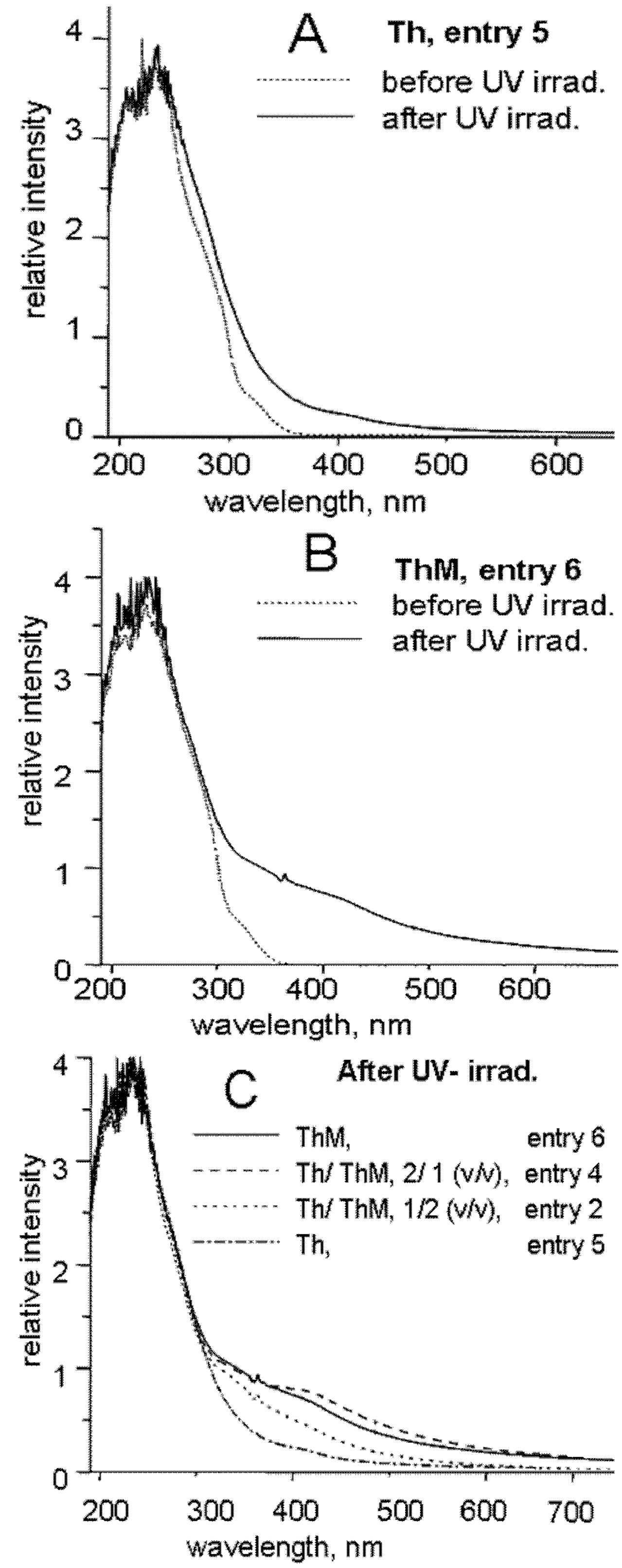
A
Th, entry 5
before UV irrad.
after UV irrad.
relative intensity
0
1
2
3
4
200
300
400
500
600
wavelength, nm
B
ThM, entry 6
before UV irrad.
after UV irrad.
relative intensity
0
1
2
3
4
200
300
400
500
600
wavelength, nm
C
After UV- irrad.
ThM, entry 6
Th/ ThM, 2/ 1 (v/v), entry 4
Th/ ThM, 1/2 (v/v), entry 2
Th, entry 5
relative intensity
0
1
2
3
4
200
300
400
500
600
700
wavelength, nm

Figure 4

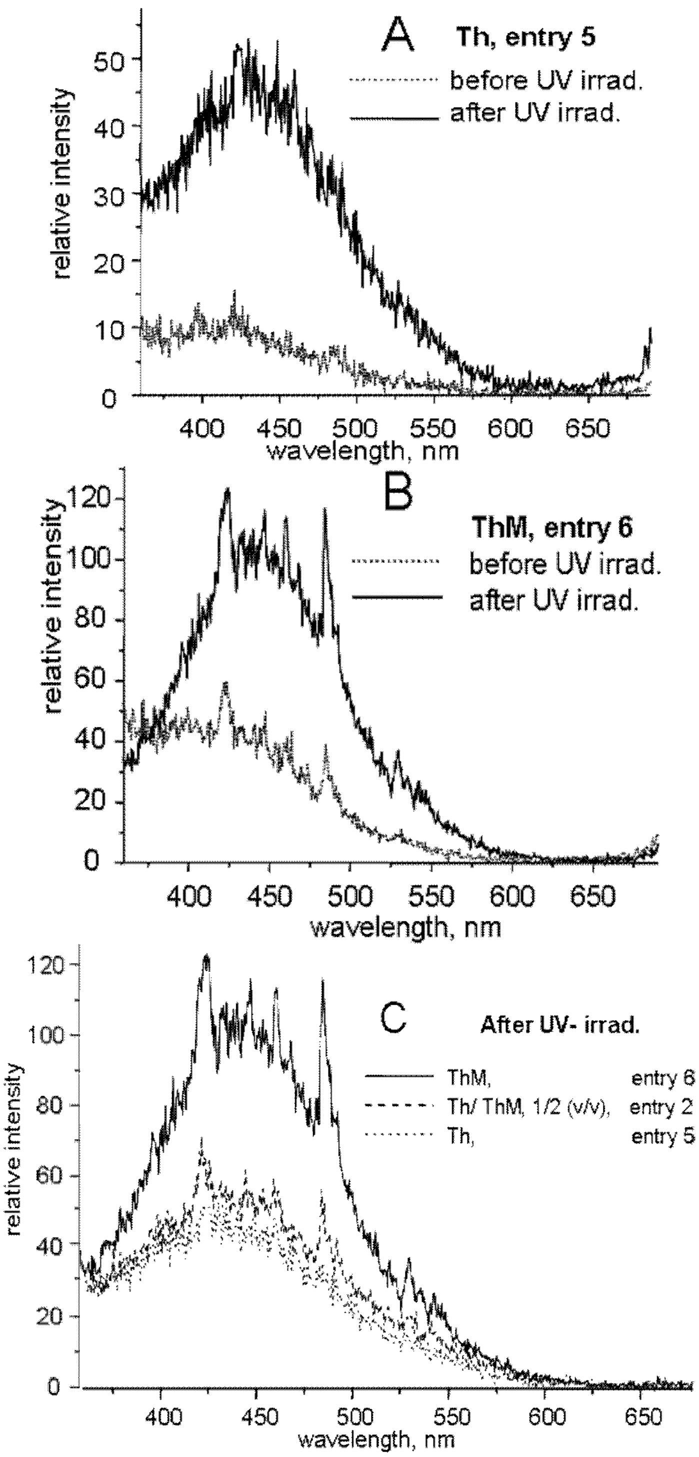
A Th, entry 5
before UV irrad.
after UV irrad.
relative intensity
wavelength, nm
B ThM, entry 6
before UV irrad.
after UV irrad.
relative intensity
wavelength, nm
C After UV- irrad.
ThM, entry 6
Th/ ThM, 1/2 (v/v), entry 2
Th, entry 5
relative intensity
wavelength, nm

Figure 5

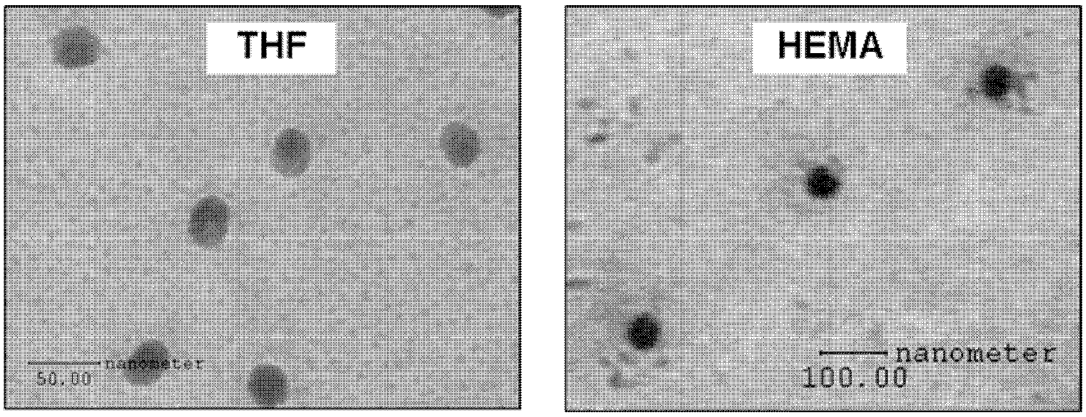


**Figure 6**

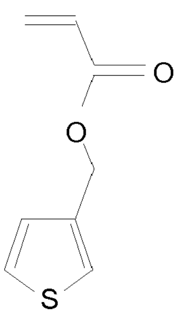

**Figure 7**

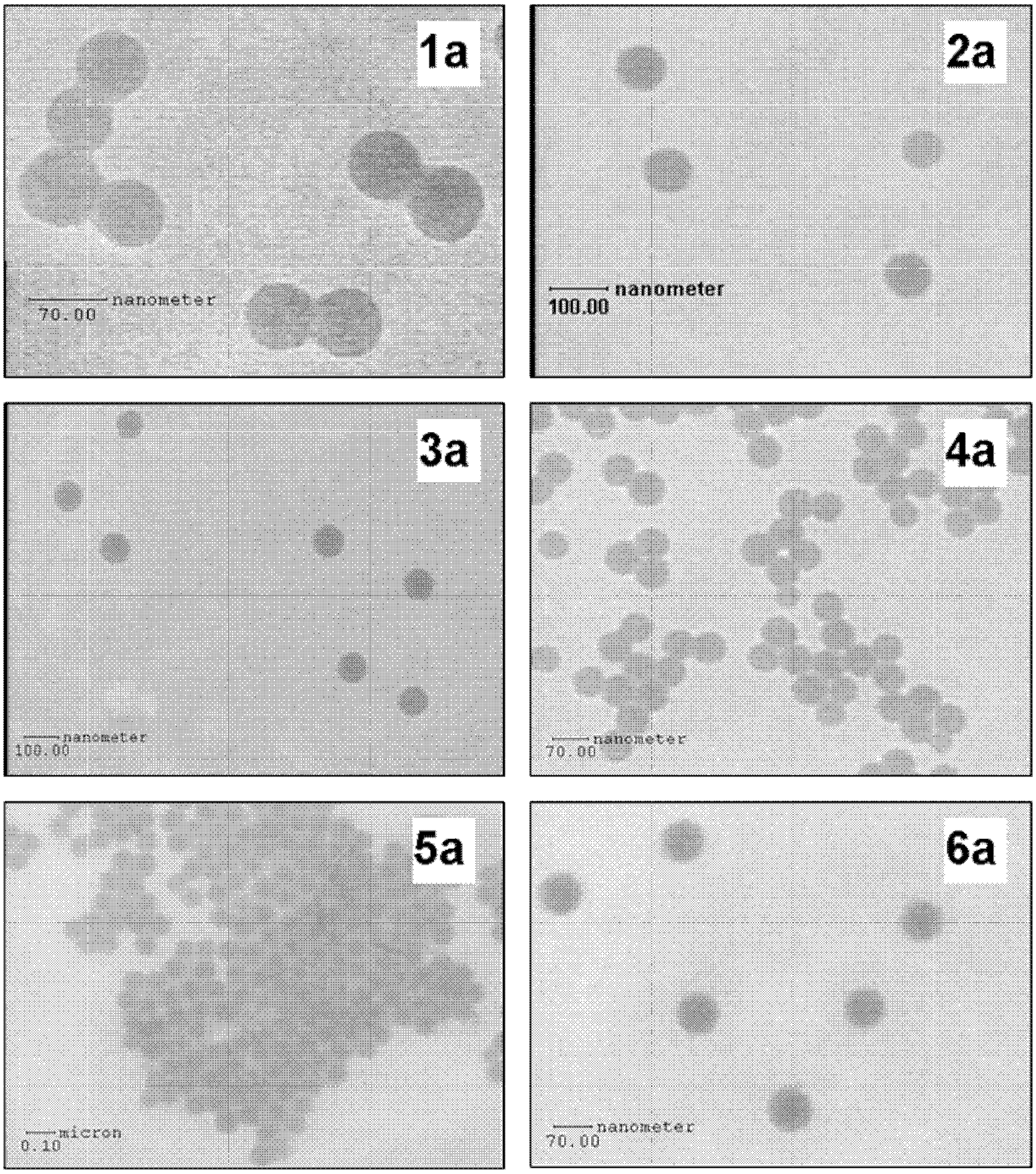
1a
nanometer
70.00
2a
nanometer
100.00
3a
nanometer
100.00
4a
nanometer
70.00
5a
micron
0.10
6a
nanometer
70.00

Figure 8

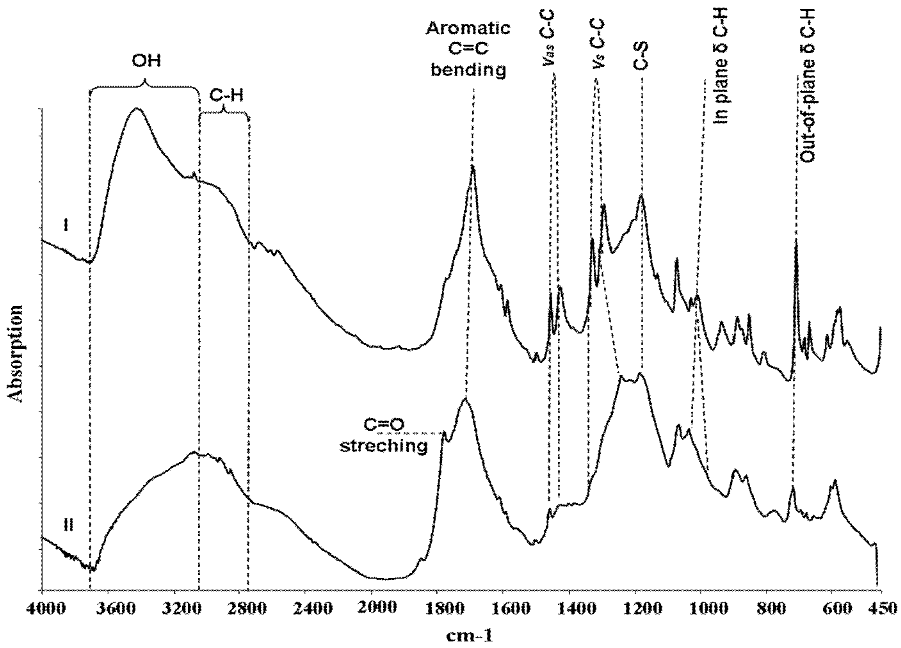


Figure 9

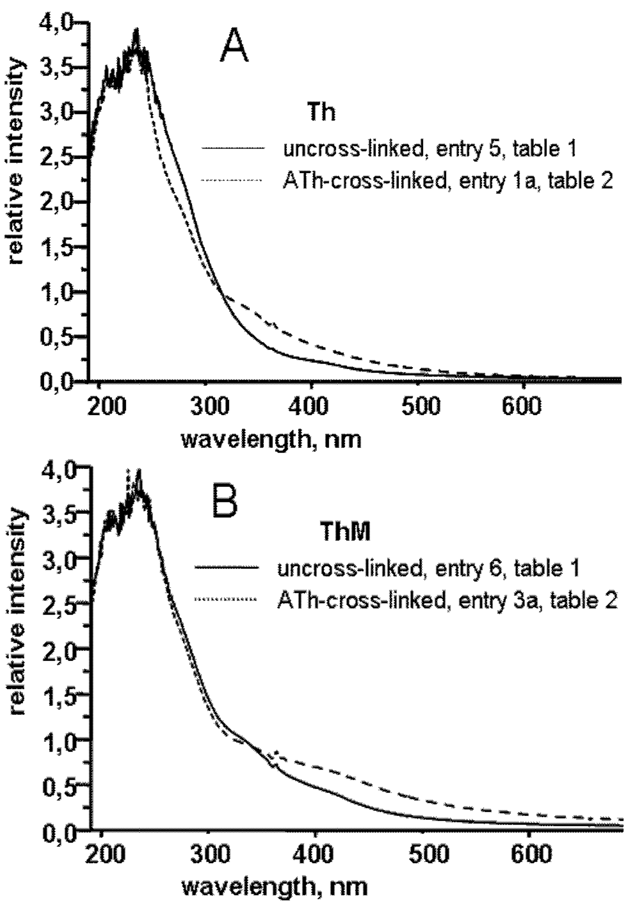


Figure 10

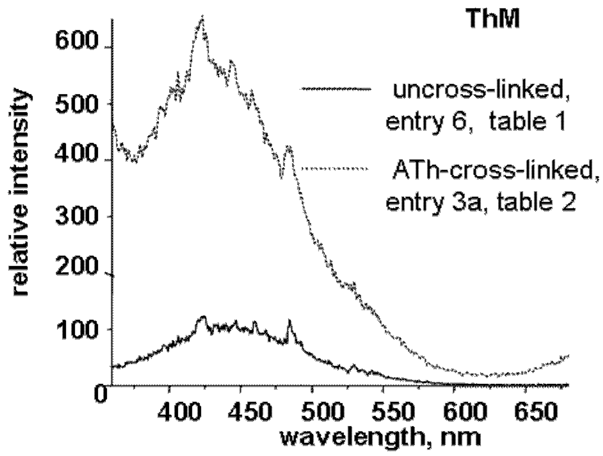


Figure 11

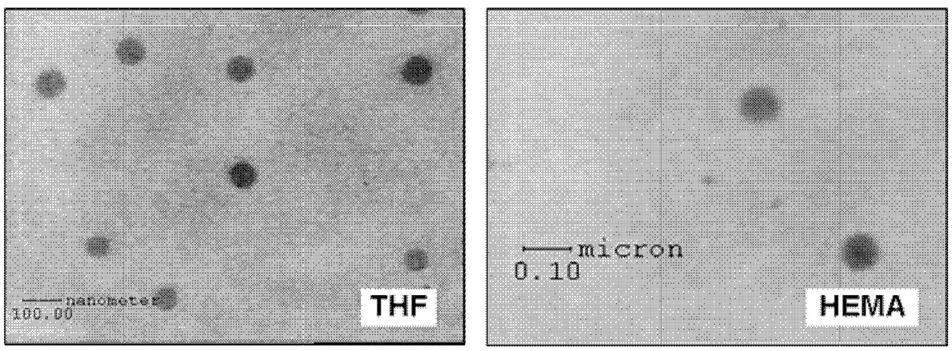


Figure 12

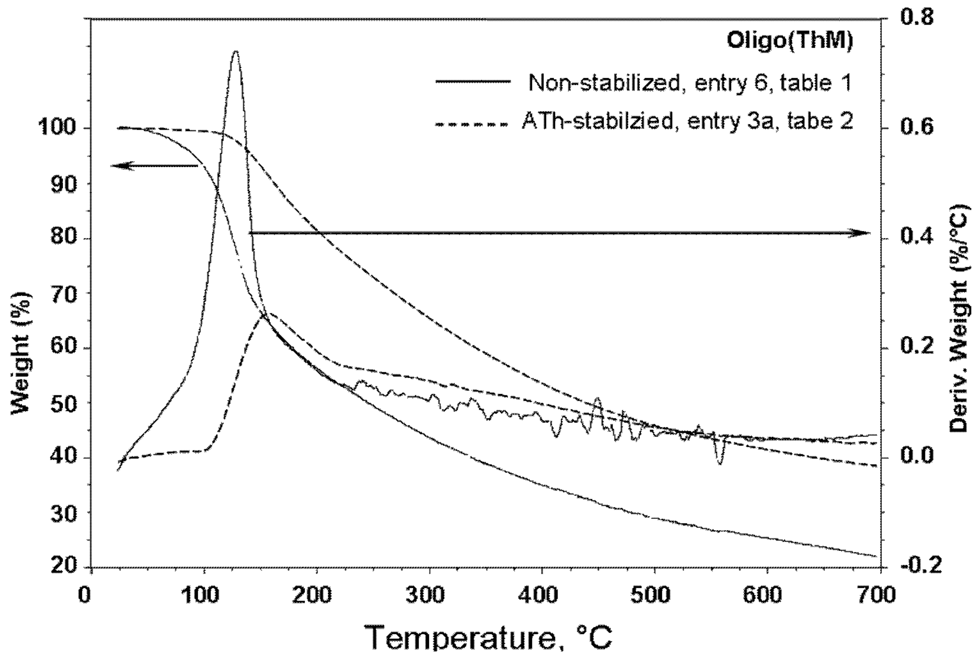


Figure 13

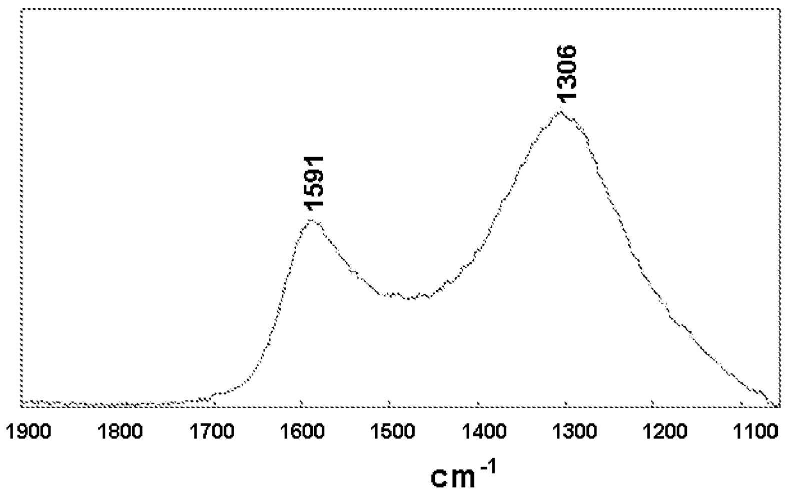


Figure 14

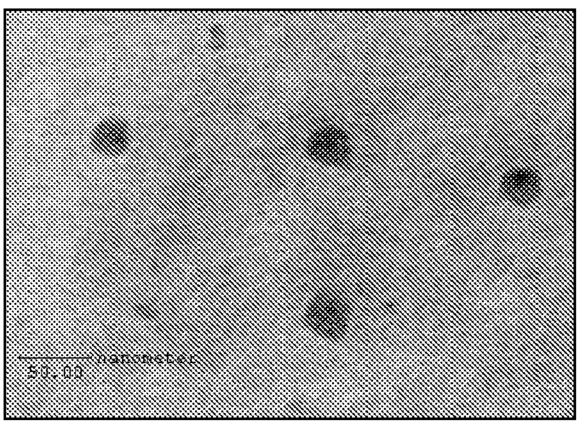


Figure 15

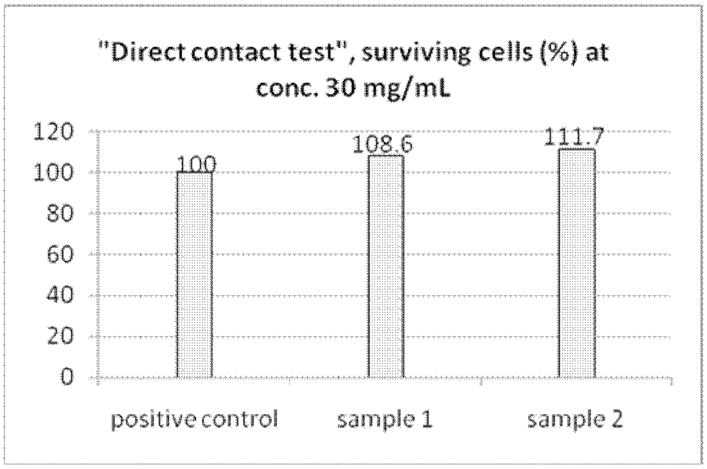


Figure 16

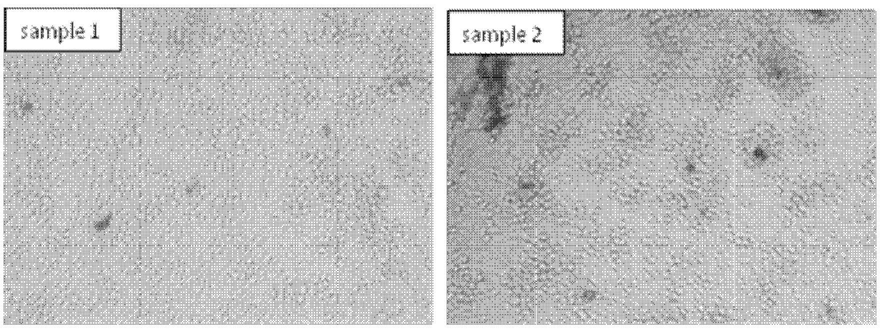


Figure 17

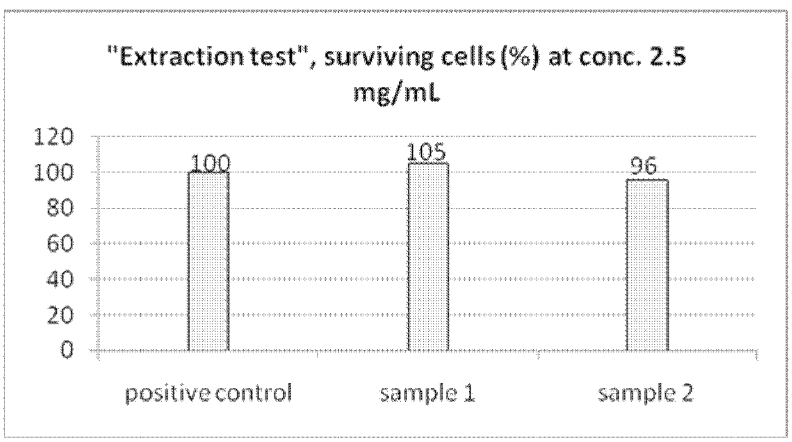


Figure 18

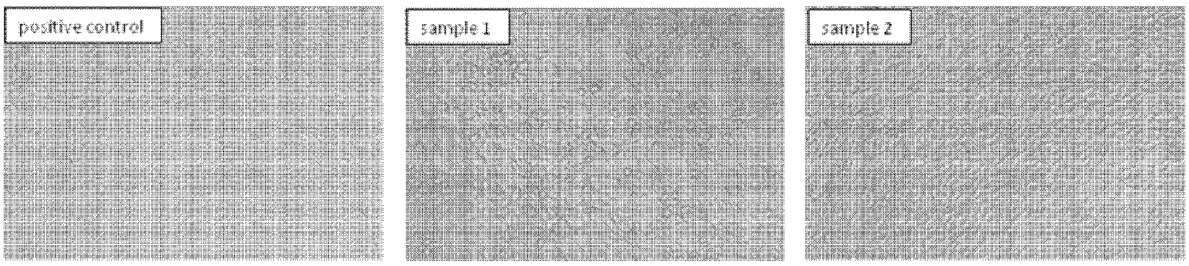


Figure 19

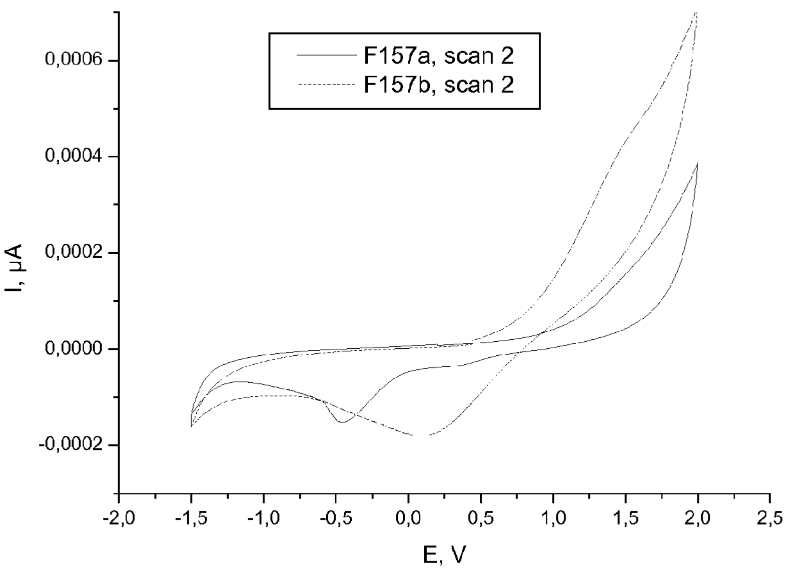


Figure 20

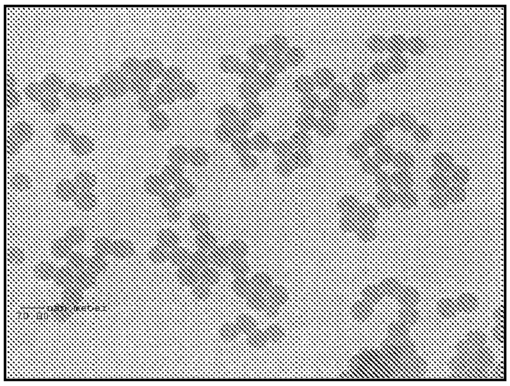

**Figure 21**

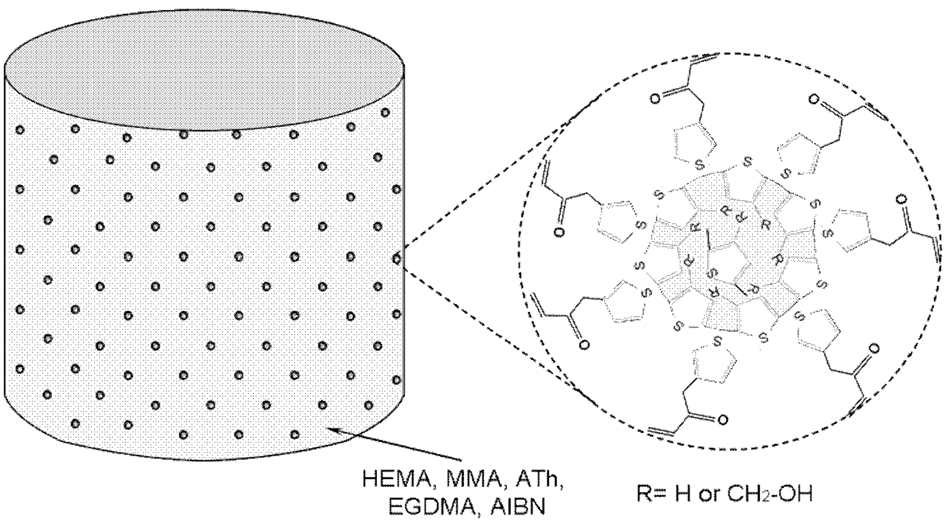


Figure 22

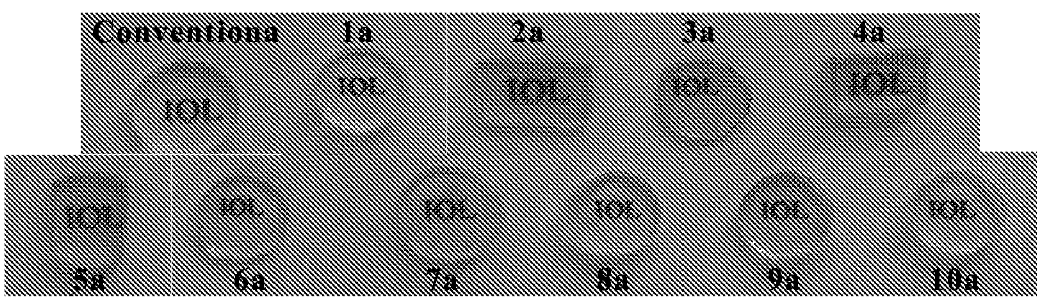


**Figure 23**

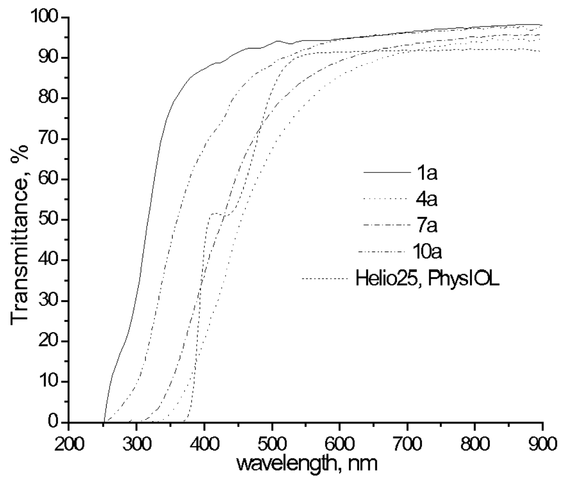


Figure 24A

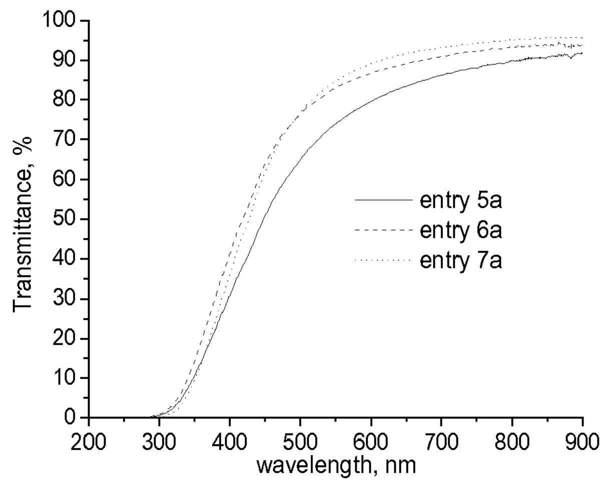


Figure 24B

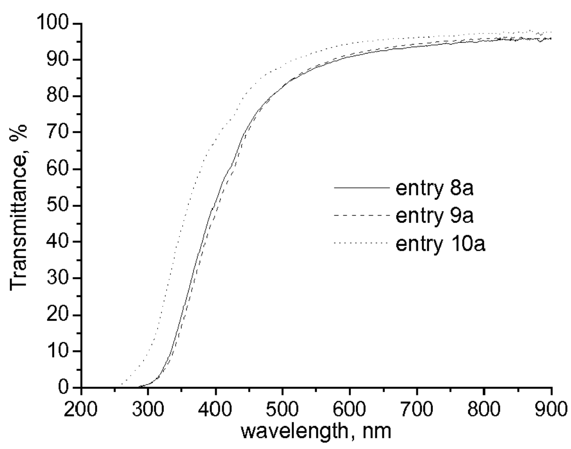


Figure 24C

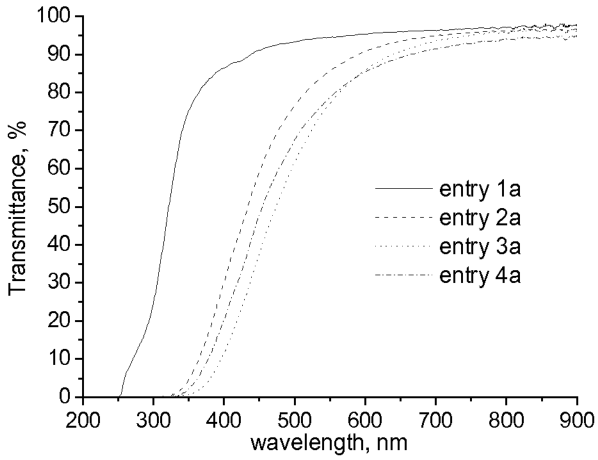


Figure 24D

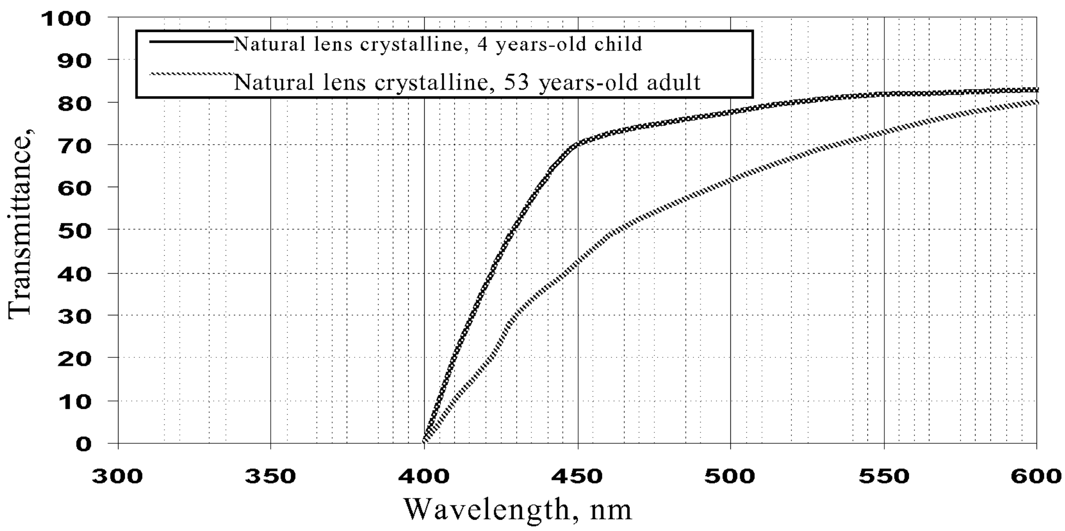


Figure 25

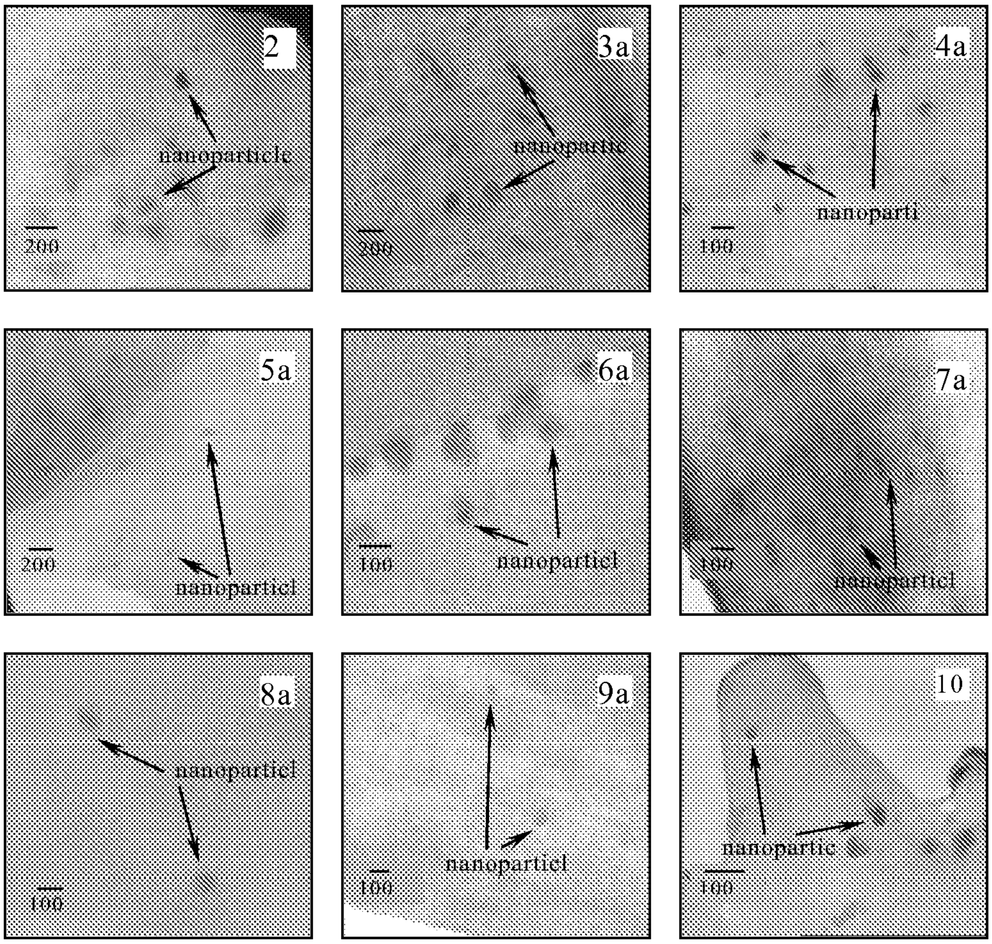
2
nanoparticle
200
3a
nanopartic
200
4a
nanoparti
100
5a
200
nanoparticl
6a
100
nanoparticl
7a
100
nanoparticl
8a
nanoparticl
100
9a
100
nanoparticl
10
nanopartic
100

Figure 26

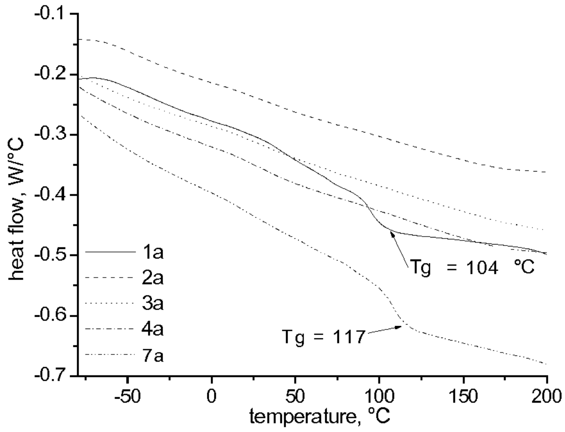


Figure 27A

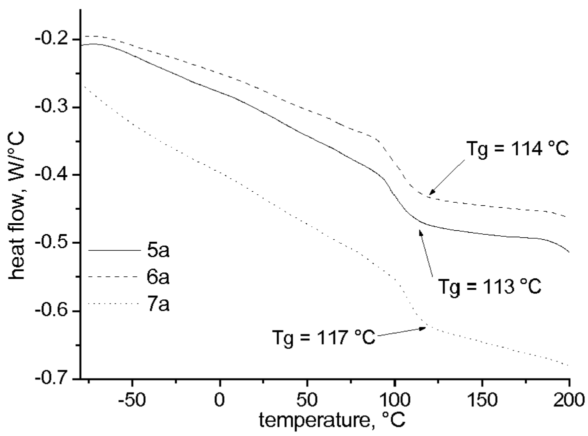


Figure 27B

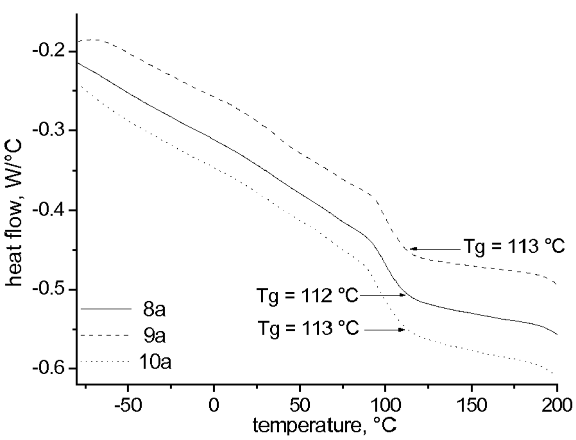


Figure 27C

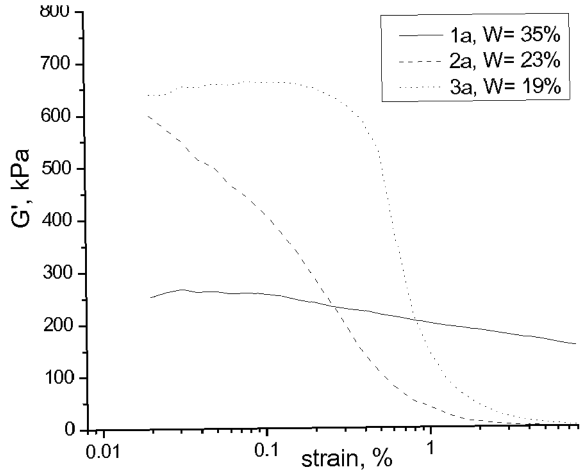


**Figure 28A**

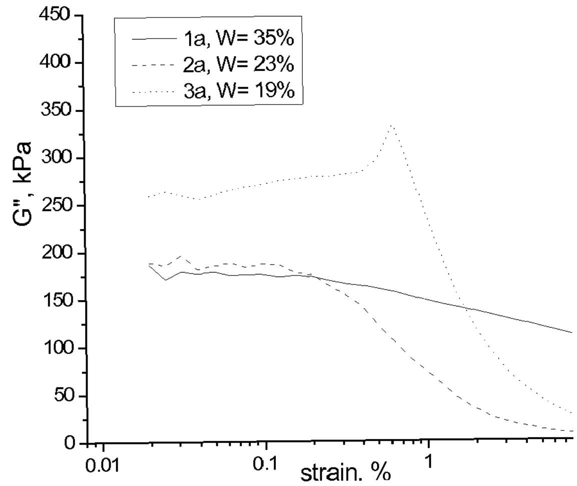


**Figure 28B**

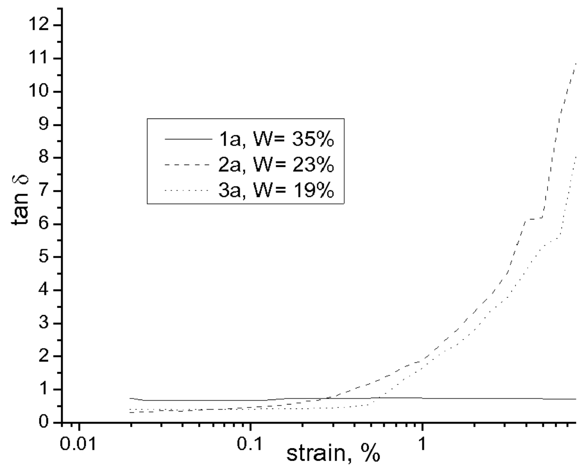


**Figure 28C**

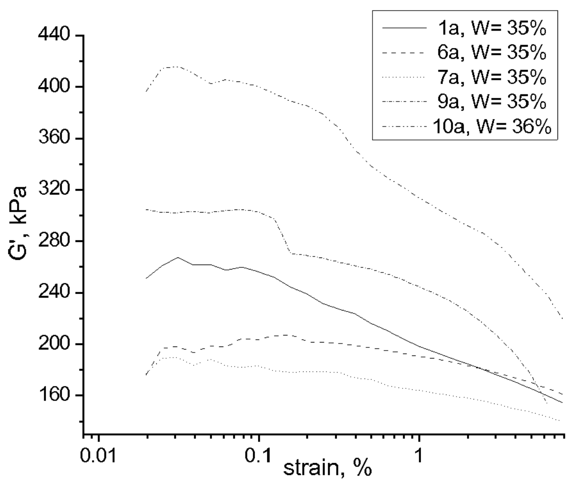


**Figure 28D**

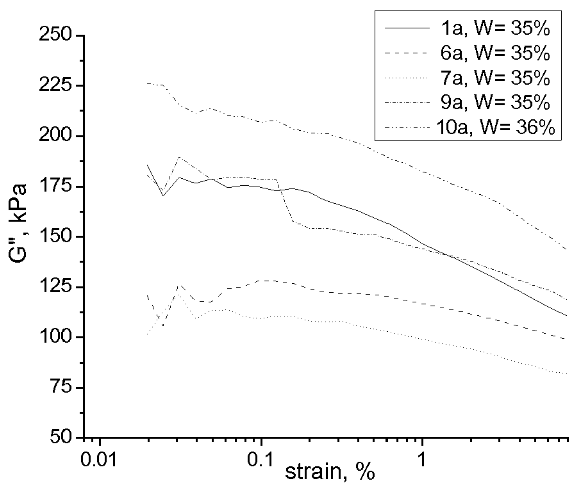


Figure 28E

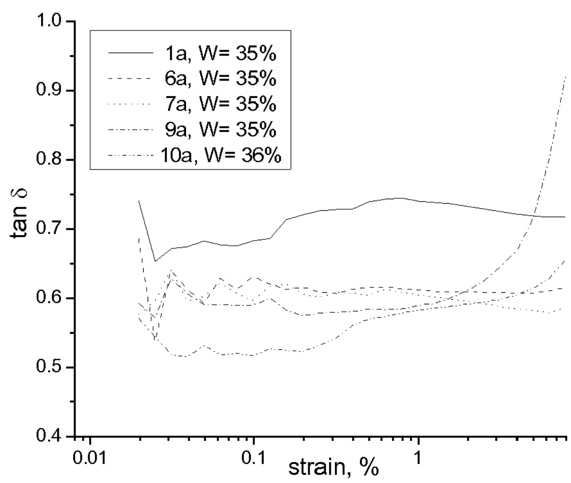


Figure 28F

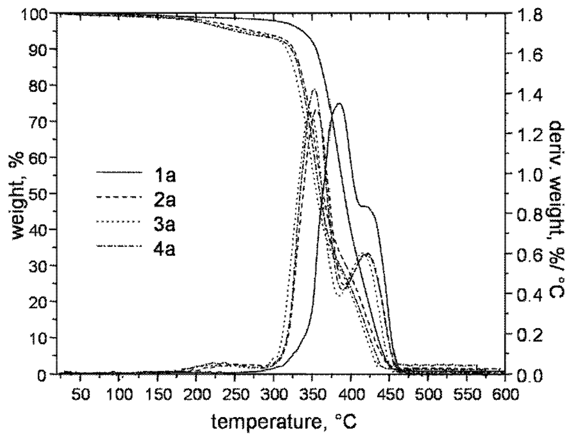


Figure 29A

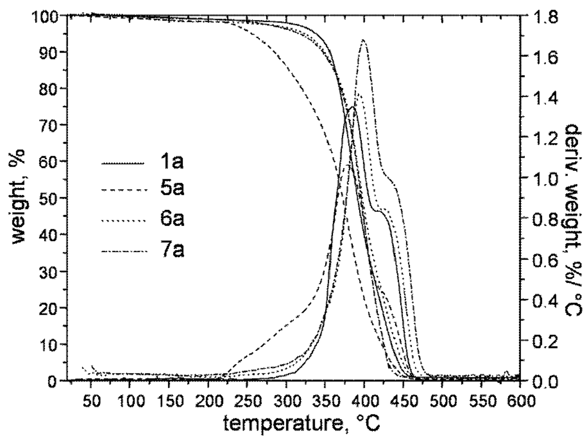


Figure 29B

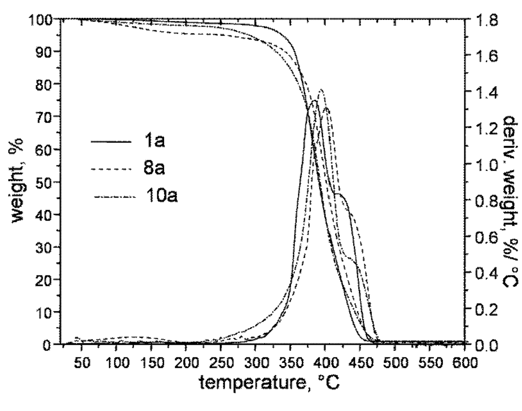


Figure 29C

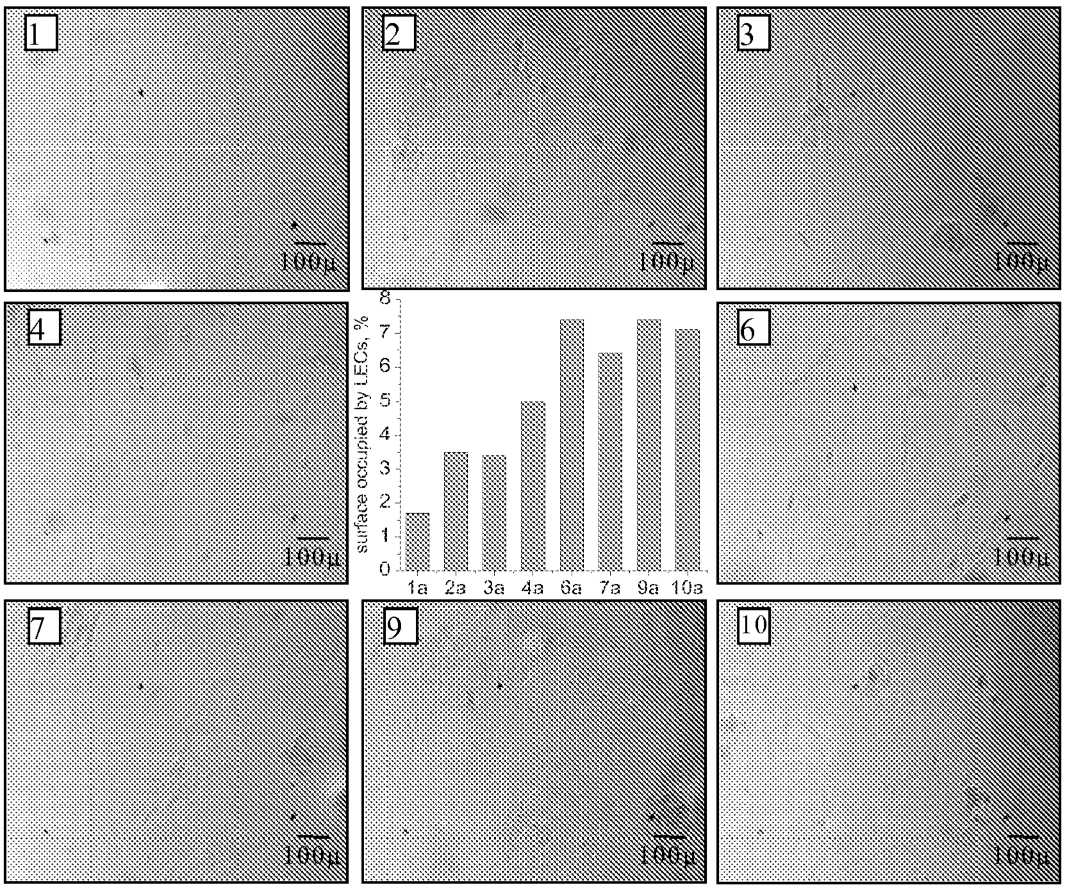
1
2
3
4
6
7
9
10
100µ
surface occupied by LECs, %
0
1
2
3
4
5
6
7
8
1a 2a 3a 4a 6a 7a 9a 10a

Figure 30

Europäisches Patentamt
European Patent Office
Office européen des brevets

# EUROPEAN SEARCH REPORT

Application Number
EP 10 19 4425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online] CHEMICAL ABSTRACTS SERVICE, COLUMBUS, OHIO, US; 9 October 2008 (2008-10-09), XU, YITING ET AL: "Method for synthesizing bioactive and biodegradable hydrogel-conductive polymer nanocomposite", XP002638028, retrieved from STN Database accession no. 2008:1216826 * abstract * -& CN 101 280 094 A (XIAMEN UNIVERSITY, PEOP. REP. CHINA) 8 October 2008 (2008-10-08) ----- | 1,2,4-9, 11-14 | INV. A61F2/16 C08G61/12 C08K5/07 A61L27/18 A61L27/48 ADD. C08L65/00 |
| X | DE BARROS R A ET AL: "Conducting polymer photopolymerization mechanism: The role of nitrate ions (NO3<->)", SYNTHETIC METALS, ELSEVIER SEQUOIA, LAUSANNE, CH, vol. 160, no. 1-2, 1 January 2010 (2010-01-01), pages 61-64, XP026817056, ISSN: 0379-6779 [retrieved on 2009-11-08] * abstract; figure 3 * ----- | 1,2,5, 7-9,12, 13 | TECHNICAL FIELDS SEARCHED (IPC) A61F C08G C08K C08L C09D A61L |

-/--

The present search report has been drawn up for all claims

| Place of search | Date of completion of the search | Examiner |
|---|---|---|
| Munich | 20 May 2011 | Meiners, Christian |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 19 4425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online] CHEMICAL ABSTRACTS SERVICE, COLUMBUS, OHIO, US; 26 October 2006 (2006-10-26), Sugita, Atsushi; Aoshima, Shinichiro; Tsuchiya, Hiroshi: "Photopolymerization method for patterned reaction products containing conducting polymers and apparatus for it", XP002638029, retrieved from STN Database accession no. 2006:1118325 * abstract * -& JP 2006 290912 A (RES FOUNDATION FOR OPTO SCIENC; UNIV SHIZUOKA NAT UNIV CORP) 26 October 2006 (2006-10-26) * paragraphs [0030] - [0038]; figures 1-3,10,11 * ----- | 1,3, 7-10,12, 13 | |
| X | FUJITSUKA, M.; SATO, T.; SEGAWA, H.; SHIMIDZU, T.: "Photochemical Polymerization of Oligothiophene and Dithienothiophene", SYNTHETIC METALS, vol. 69, 1 March 1995 (1995-03-01), pages 309-310, XP002638030, * abstract; figures 1,2 * ----- | 1,3,7, 12,13 | TECHNICAL FIELDS SEARCHED (IPC) |
| | -/-- | | |

The present search report has been drawn up for all claims

| Place of search | Date of completion of the search | Examiner |
|---|---|---|
| Munich | 20 May 2011 | Meiners, Christian |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 19 4425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAZQUEZ M ET AL: "Solution-cast films of poly(3,4-ethylenedioxythiophene) as ion-to-electron transducers in all-solid-state ion-selective electrodes", SENSORS AND ACTUATORS B, ELSEVIER SEQUOIA S.A., LAUSANNE, CH, vol. 97, no. 2-3, 1 February 2004 (2004-02-01), pages 182-189, XP004486616, ISSN: 0925-4005, DOI: DOI:10.1016/J.SNB.2003.08.010 * abstract * ----- | 7-13 | |
| X | DISPENZA C ET AL: "Electrically conductive hydrogel composites made of polyaniline nanoparticles and poly(N-vinyl-2-pyrrolidone)", POLYMER, ELSEVIER SCIENCE PUBLISHERS B.V, GB, vol. 47, no. 4, 8 February 2006 (2006-02-08), pages 961-971, XP025231387, ISSN: 0032-3861, DOI: DOI:10.1016/J.POLYMER.2005.12.071 [retrieved on 2006-02-08] * abstract; figures 4,5 * ----- | 7-9, 11-13 | TECHNICAL FIELDS SEARCHED (IPC) |
| X | WO 2010/015468 A1 (H C STARCK CLEVIOS GMBH [DE]; LOEVENICH WILFRIED [DE]; KARABULUT HIKME) 11 February 2010 (2010-02-11) * abstract * * claims 1,7,9 * ----- | 7-13 | |
| X | US 2007/207182 A1 (WEBER JAN [US] ET AL) 6 September 2007 (2007-09-06) * paragraphs [0046], [0105] * ----- | 7-9, 11-16 | |
| | -/-- | | |

The present search report has been drawn up for all claims

| Place of search | Date of completion of the search | Examiner |
|---|---|---|
| Munich | 20 May 2011 | Meiners, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 10 19 4425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KOCK T J J M ET AL: "A radiation-crosslinkable thiophene copolymer", SYNTHETIC METALS, ELSEVIER SEQUOIA, LAUSANNE, CH, vol. 79, no. 3, 30 May 1996 (1996-05-30), pages 215-218, XP027394424, ISSN: 0379-6779 [retrieved on 1996-05-30] * abstract; figure 1 * ----- | 7-10,12, 13 | |
| X | DATABASE CA [Online] CHEMICAL ABSTRACTS SERVICE, COLUMBUS, OHIO, US; 17 February 1990 (1990-02-17), Shimizu, T.; Honda, K.; Yada, T.; Segawa, K.: "Formation of electrically conductive pattern by photopolymerisation", XP002638031, Database accession no. 1990:66560 * abstract * -& JP 1 123228 A (RESEARCH DEVELOPMENT CORP. OF JAPAN, JAPAN) 16 May 1989 (1989-05-16) ----- | 1-3,5, 7-9,12, 13 | TECHNICAL FIELDS SEARCHED (IPC) |
| X | DATABASE CA [Online] CHEMICAL ABSTRACTS SERVICE, COLUMBUS, OHIO, US; 19 January 2006 (2006-01-19), YAMADA, KATSUMI ET AL: "Micropattern formation of electroconductive polymers by multiphoton-absorption-stimulated polymerization using laser beams", XP002638032, retrieved from STN Database accession no. 2006:50953 * abstract * -& JP 2006 016582 A (JAPAN) 19 January 2006 (2006-01-19) ----- | 1-13 | |

The present search report has been drawn up for all claims

| Place of search | Date of completion of the search | Examiner |
|---|---|---|
| Munich | 20 May 2011 | Meiners, Christian |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 19 4425

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2011

| Patent document cited in search report | Publication date | Patent family member(s) | Publication date |
|---|---|---|---|
| CN 101280094 A | 08-10-2008 | NONE | |
| JP 2006290912 A | 26-10-2006 | NONE | |
| WO 2010015468 A1 | 11-02-2010 | DE 102008036525 A1 | 11-02-2010 |
| US 2007207182 A1 | 06-09-2007 | CA 2645049 A1 | 13-09-2007 |
| | | EP 2010240 A2 | 07-01-2009 |
| | | JP 2009528895 T | 13-08-2009 |
| | | WO 2007103356 A2 | 13-09-2007 |
| JP 1123228 A | 16-05-1989 | JP 2676515 B2 | 17-11-1997 |
| JP 2006016582 A | 19-01-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHOI et al.** *Synth. Mater.,* 2004, vol. 141, 293-299
- **LANDFESTER et al.** *Adv. Mater.,* 2002, vol. 14/9, 651-655
- **XIA et al.** *J. Am. Chem. Soc.,* 2004, vol. 126, 8735-8743
- **ZHAI, L. et al.** *J. Mater. Chem.,* 2004, vol. 14, 141-143
- **SI et al.** *J. Am. Chem. Soc.,* 2007, vol. 129, 3888-3896
- **ZHANG, Z. et al.** *Mater. Lett.,* 2006, vol. 60, 1039-1042
- **SURIN et al.** *Synth. Met.,* 2004, vol. 147, 67-72
- **ONG et al.** *Adv. Mater.,* 2005, vol. 17, 1141-1144
- **ADVINCULA.** *Dalton Trans.,* 2006, 2778-2784
- **LEE et al.** *Current Appl. Phys.,* 2008, 659-663
- **OH et al.** *Current Appl. Phys.,* 2002, vol. 2, 273-277
- **KOCK et al.** *Synth. Metals.,* 1996, vol. 79, 215-218
- **GUNER.** *Europ. Polym. J.,* 2004, vol. 40, 1799-1806
- **BOZUKOVA et al.** *Biomacromolecules,* 2007, vol. 8, 2379-2387
- **QIAO et al.** *Carbon,* 2006, vol. 44 (1), 187-190
- **LI et al.** *Polymer,* 2007, vol. 48, 3982-3989
- **MEAKIN et al.** *J. Mater. Sci.: Mater. Medic.,* 2003, vol. 14, 783-787
- **ANSETH et al.** *Biomaterials,* 1996, vol. 17, 1647-1657
- **MEAKIN et al.** *J. Mater. Sci. Mater. Medic.,* 2003, vol. 14, 783-787